(19) Europäisches Patentamt
European Patent Office
Office européen des brevets

(11) Numéro de publication: **0 089 290 B1**

(12) # FASCICULE DE BREVET EUROPEEN

(45) Date de publication du fascicule du brevet: **14.06.89**

(51) Int. Cl.⁴: **A 61 K 39/385**

(21) Numéro de dépôt: **83400525.8**

(22) Date de dépôt: **15.03.83**

(54) **Conjugués d'haptènes et de muramyl-peptides, doués d'activité immunogène et compositions les contenant.**

(30) Priorité: **15.03.82 FR 8204353**

(43) Date de publication de la demande:
**21.09.83 Bulletin 83/38**

(45) Mention de la délivrance du brevet:
**14.06.89 Bulletin 89/24**

(84) Etats contractants désignés:
**AT BE CH DE FR GB IT LI LU NL SE**

(56) Documents cités:
**EP-A- 0 003 833**
**FR-A- 2 292 486**

**CHEMICAL ABSTRACTS, vol. 93, no. 19, 10 novembre 1980, page 474, no. 184067b, Columbus, Ohio, USA**
**Dictionnaire de Chimie et de ses applications, 3e Ed., p. 515**

(73) Titulaire: **ANVAR Agence Nationale de Valorisation de la Recherche, 43, rue Caumartin, F-75436 Paris Cédex 09 (FR)**

(72) Inventeur: **Audibert, Françoise, 44, rue Ybry, F-92200 Neuilly sur Seine (FR)**
Inventeur: **Carelli, Claude, 64, rue Rouget de l'Isle, F-92150 Suresnes (FR)**
Inventeur: **Chedid, Louis, 16-18, rue Gaston de Caillavet, F-75015 Paris (FR)**
Inventeur: **Lefrancier, Pierre, 46, Allée de la Mare l'Oiseau, F-91190 Gif sur Yvette (FR)**
Inventeur: **Level, Michel, 24-26, rue Sibuet, B.62, F-75012 Paris (FR)**
Inventeur: **Choay, Jean, 21, rue Saint Guillaume, F-75007 Paris (FR)**

(74) Mandataire: **Gutmann, Ernest et al, S.C. Ernest Gutmann - Yves Plasseraud 67, boulevard Haussmann, F-75008 Paris (FR)**

EP 0 089 290 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention est relative à une nouvelle catégorie de composés, dérivés de molécules de petit poids moléculaire et modifiés par des dérivés de muramyl-peptide, cette modification étant apte à leur conférer des propriétés d'immunogénicité dont ils étaient essentiellement dépourvus, avant leur modification par ces muramyl-peptides.

On sait que les dérivés communément dénommés «muramyl-peptides» et qui comportent un motif saccharidique, notamment du type N-acétyl-muramyle, et une chaîne peptidique rattachée à ce motif saccharidique, sont connus comme possédant des propriétés d'adjuvants immunologiques. De nombreux représentants de ces muramyl-peptides ont déjà été décrits dans la littérature technique.

La capacité qu'ont les muramyl-peptides de stimuler la réponse immunitaire d'un hôte à l'égard d'un antigène permet d'envisager leur application, par exemple à des compositions vaccinantes mettant en jeu un agent immunogène, soit faible par nature, soit affaibli, en raison de purifications poussées, notamment en raison de l'existence de toxicité ou d'autres effets secondaires néfastes dans l'antigène initial.

Pour renforcer davantage encore l'effet immunologique adjuvant des muramyl-peptides, il a encore été proposé, notamment dans la demande de brevet européen EP-A-3 833, de fixer ces dérivés de muramyl-peptides sur les antigènes étudiés, notamment par l'intermédiaire de liaisons covalentes, en d'autres termes de les «conjuguer» à ces antigènes. Dans certains cas, ce type de liaison permet une potentialisation supplémentaire de l'effet immunogène de l'antigène ainsi modifié. Lorsque l'antigène présente un poids moléculaire faible, il est recommandé, selon le brevet européen EP-A-3 833 de le conjuguer également à une molécule porteuse de poids moléculaire plus élevé. Il a cependant été observé également que cette conjugation pouvait s'accompagner de propriétés pyrogènes et de réactions immunogènes distinctes, plus particulièrement dirigées contre le dérivé de muramyl-peptide lui-même. C'est, à titre d'exemple, ce que l'on observe avec des conjugués réalisés entre la toxine tétanique et des dérivés de muramyl-peptide. La production d'un conjugué formé entre l'eicosapeptide de C-terminal de la bêta-hCG et d'un muramyl-peptide a également été envisagée dans ce brevet européen. Ses propriétés n'on pas été indiquées.

Parallèlement à ces techniques, on a vu se développer un nouveau type d'agents immunogènes consistant en des produits de conjuguaison entre un fragment possédant la structure d'une partie d'un antigène naturel ou «natif» et une molécule support, notamment une macromolécule naturelle ou synthétique. Par exemple, le fragment consiste en un peptide possédant une séquence commune avec un antigène peptidique natif. Cependant, à l'exception de quelques peptides qui possédaient déjà en eux-mêmes un nombre d'acides aminés suffisant pour leur conférer une activité immunogène extrêmement faible, mais décelable, l'immunogénicité de ces fragments est en général tributaire de leur conjugaison avec ledit support.

On se référera par exemple aux publications récentes suivantes qui décrivent de tels principes immunogènes:

– AUDIBERT, JOLIVET, CHEDID, ALOUF, BOQUET, RIVAILLE et SIFFERT, «Nature», vol. 289, No. 5798, p. 593–594, 12 février 1981,

– MOZES, SELA et CHEDID, «Proc. Natl. Acad. Sci. USA», vol. 77, No. 8, p. 4933–4937, août 1980,

– ARNON, SELA, PARANT et CHEDID, «Proc. Natl. Acad. Sci. USA», vol. 77, No. 11, p. 6769–6772, novembre 1980,

– LERNER, GREEN, ALEXANDER, LIU, SUTCLIFFE et SHINNICK, «Proc. Natl. Acad. Sci. USA», vol. 78, No. 6, p. 3403–3407, juin 1981,

– ARNON, MARON, SELA et ANFINSEN, «Proc. Natl. Acad. Sci. USA», vol. 68, No. 7, p. 1450–1455, juillet 1981, et

– SHUJI MATSUURA, MASANOBU OHASHI, HAO-CHIA CHEN et Gary D. HODGEN, «Endocrinology», vol. 104, No. 2, 1979.

Certains auteurs ont déjà testé la capacité de certains peptides d'induire la production d'anticorps en l'absence de tout couplage préalable avec une macromolécule support. Par exemple, R.A. LERNER et al. dans le document déjà cité et G.R. DREESMAN («Nature», vol. 298, du 14 janvier 1982, p. 158–160) constatent que certains peptides ayant des séquences en commun avec l'antigène HBsAg ont conduit à la production d'anticorps détectables chez la souris. Selon DREESMAN, l'association de ces peptides avec un adjuvant, plus particulièrement l'adjuvant complet de Freund (FCA), un alun, des liposomes, auxquels a éventuellement été incorporé le N-acétyl-muramyl-L-alanyl-D-isoglutamine (MDP) n'a pas eu pour effet d'accroître de façon significative la réponse immunitaire primaire induite par les peptides du genre en question.

L'invention découle de la découverte que le couplage, essentiellement en l'absence de macromolécule support, à un haptène ou fragment haptènique de faible poids moléculaire possédant au moins un «site antigénique», par liaison covalente du MDP ou d'analogues, homologues ou dérivés de celui-ci, en particulier de tous dérivés muramyl-peptides ou analogues connus pour posséder des propriétés adjuvantes de la réponse immunitaire à l'égard d'antigènes, conduisant à des composés immunogènes ayant une efficacité immunogénique inattendue in vivo.

Ces immunogènes présentent en outre la qualité inattendue d'être dépourvus pratiquement ou totalement d'effet pyrogène in vivo, contrairement aux conjugués préalablement décrits entre le MDP et des antigènes de masse moléculaire élevée (ceux-ci consistant en un antigène naturel ou en un conjugué d'une molécule, notamment d'un peptide portant un site antigénique, à une macromolécule support). Cette absence d'effet pyrogène in vivo est constatée tant pour les dérivés ou

analogues du MDP qui sont pyrogènes lorsqu'ils sont administrés seuls et dont la pyrogénicité diminue, voire disparaît après couplage à l'haptène, que pour des dérivés ou analogues du MDP qui sont sans effet pyrogène lorsqu'ils sont administrés seuls et dont l'apyrogénicité se maintient après conjuguaison.

L'invention concerne plus particulièrement le conjugué ayant un effet immunogène entre un haptène possédant au moins un site antigénique et un muramyl-peptide, ou dérivé de muramylpeptide, caractérisé par l'absence de macromolécule support, ce conjugué étant encore davantage caractérisé en ce que la partie haptène présente un poids moléculaire inférieur à 5000 pour annuler l'effet pyrogène qui est susceptible d'être observé à l'occasion du couplage de muramyl-peptides sur des antigènes de poids moléculaire plus élevé et, qu'en outre, ce conjugué présente un poids moléculaire inférieur à 5000 pour que le conjugué obtenu ne soit pas apte à induire en sus de l'immunogénicité attendue, la production d'anticorps dirigés contre la partie muramyl-peptide elle-même du conjugué, avec la condition supplémentaire que l'haptène est différent de l'eicosapeptide C-terminal de la bêta-hCG.

Dans ce qui précède, on donne au terme «haptène» un sens très large. Il signifie tout haptène ou fragment hapténique de faible poids moléculaire, quel que soit son mode d'obtention, qu'il s'agisse de fractionnement, de dépolymérisation, de synthèse ou de recombinaison génétique.

Egalement dans ce qui précède, l'expression «site antigénique» signifie toute configuration, notamment de surface, de l'haptène susceptible d'être reconnue par un anticorps préalablement formé à l'égard d'un antigène ayant un site en commun avec cet haptène. L'haptène dont la conformation peut être modifiée par la conjuguaison au produit de type MDP, conformément à l'invention, induit in vivo des anticorps ou une réaction spécifique à médiation cellulaire, non seulement contre lui-même, mais en outre – dans le cas où l'haptène a un élément de structure en commun avec un antigène natif – contre l'antigène natif lui-même ou l'antigène que l'on peut éventuellement former par couplage de l'haptène avec un support macromoléculaire. Par exemple, un haptène consistant en une séquence comprenant un nombre réduit d'acides aminés est, quand il est couplé à un muramyl-peptide, capable d'induire in vivo la formation d'anticorps actifs contre le polypeptide ou la protéine antigénique ayant en commun avec l'haptène la même séquence d'acides aminés.

Il en est naturellement de même en ce qui concerne des haptènes non exclusivement peptidiques. A cet égard, l'haptène consiste par exemple en une structure osidique de poids moléculaire réduit, pouvant être constituée d'un monosaccharide ou de plusieurs monosaccharides liés entre eux par exemple par une liaison de type glycosidique. Ces oligosaccharides peuvent être soit linéaires, soit ramifiés. Ils peuvent appartenir à la famille des hexoses ou des pentoses; ils peuvent être neutres ou aminés, contenir un acide uronique ou un acide sialique. Ils peuvent porter des substituants usuellement contenus dans les structures naturelles, tels que acétyle, sulfate ou phosphate. Ces indications sont données à titre d'exemple mais ne sont pas limitatives et l'on envisage d'une manière générale tous les haptènes peptidiques et osidiques dont on retrouve les équivalents à l'intérieur de la structure d'un antigène de masse moléculaire élevée, que cet haptène soit inclus dans la chaîne ou qu'il figure en position terminale.

L'invention concerne plus particulièrement les conjugués de muramyl-peptide et d'haptène appartenant aux catégories déjà évoquées ci-dessus qui, lorsqu'ils sont administrés in vivo, induisent une réaction immunitaire (production d'anticorps ou réaction à médiation cellulaire) à l'égard de l'antigène natif contenant un élément de structure commun avec l'hapène du genre en question. Il est entendu que, lorsque dans la suite il sera fait référence à la capacité des haptènes selon l'invention d'induire la production d'anticorps, il s'agit d'une simplification de langage dont le caractère n'est pas limitatif. Il est naturellement à comprendre que, de façon générale, cette expression englobera toute réaction immunitaire, y compris les réactions à médiation cellulaire.

D'une façon générale, les haptènes susceptibles d'être mis en œuvre pour la constitution des conjugués selon l'invention ont en commun un élément de structure, dans les conditions qui ont été évoquées ci-dessus, avec tout type d'antigène connu ou susceptible d'être utilisé comme principe vaccinant. On mentionnera, à titre d'exemple des antigènes en question, des principes actifs extraits de micro-organismes infectieux ou les micro-organismes infectieux eux-mêmes (vaccins atténués ou tués). Plus particulièrement, les haptènes susceptibles d'être mis en œuvre dans le cadre de l'invention peuvent avoir en commun des éléments de structure avec des constituants de ces agents pathogènes (tels que bactéries, virus parasites, rickettsies et protozoaires). A titre d'exemple, on mentionnera les haptènes ayant des éléments de structure en commun avec des protéines antigéniques appartenant aux enveloppes des virus de l'hépatite virale B, de la grippe, des virus herpétiques, ou encore des protéines de parois bactériennes, par exemple streptococciques. Au titre des haptènes ayant des éléments de structure communs avec des antigènes non exclusivement peptidiques ou non peptidiques, on peut mentionner des oligosaccharides, glycopeptides, lipopeptides, glycolipides, lipides, stéroïdes. De façon plus générale, on peut aussi se reporter aux indications fournies dans la demande de brevet européen EP-A-3 833.

L'invention s'étend aussi particulièrement aux haptènes ou fragments hapténiques de faible poids moléculaire qui peuvent être obtenus par synthèse.

L'invention concerne également des conjugués de muramyl-peptides et d'autres molécules ou fragments de molécules, telles que des hormones, notamment des hormones peptidiques, par exemple des hormones susceptibles de réguler la pression artérielle ou régulant des activités des différentes glandes endocrines, telles que hypophyse, glandes sexuelles, ou des hormones stéroïdiques, ou des neuromédiateurs, dont les neuropeptides, ou des déterminants d'antigènes tissulaires, par exemple des déterminants antigéniques de groupes sanguins, ou des lymphokines, ou des récepteurs cellulaires, qui sont naturellement présents dans les organismes vivants et immunologiquement tolérés par ceux-ci.

L'invention s'intéresse donc plus particulièrement aux conjugués de molécules déterminées ou fragments de molécules déterminées avec des dérivés de MDP, dont les propriétés immunogènes acquises leur permettront l'induction de la modification du comportement immunitaire des organismes auxquels ces conjugués sont administrés, de façon à plus particulièrement promouvoir chez ces organismes la production d'anticorps actifs contre lesdites molécules déterminées ou fragments de molécules déterminées eux-mêmes. On sait qu'il est des domaines où une telle modification est recherchée, par exemple au niveau des hormones intervenant dans la reproduction des espèces vivantes. Les molécules déterminées ou fragments de molécules déterminées peuvent être des molécules naturelles ou être issus de molécules naturelles. A cet égard, l'invention porte sur des hormones modifiées qui peuvent être éventuellement utilisées comme moyen contraceptif.

Le procédé permet aussi de réaliser une hypophysectomie immunologique sélective, laissant intactes des hormones hypophysaires ayant une grande importance pour les fonctions vitales de l'organisme, telles qu'hormone de croissance, ACTH, TSH, ainsi que les hormones de la posthypophyse. L'utilisation de cette hypophysectomie sélective et non chirurgicale peut avoir des indications cliniques importantes, notamment dans le cas de certaines tumeurs hormones hypophysaires ou gonadotropines dépendantes.

L'invention s'intéresse de façon plus immédiate encore à des hormones modifiées conformément à l'invention, susceptibles d'être utilisées comme vaccins vétérinaires permettant un accroissement de la production de viande par castration immunologique.

L'intérêt du procédé réside aussi en ce que les hormones dépendantes de LH-RH sont importantes pour le bon développement de l'animal et sa prise de poids jusqu'à plusieurs mois (6 à 9 mois), âge auquel il n'est plus pratiquement possible de procéder à la castration chirurgicale, ce qui explique que celle-ci soit pratiquée avant 3 mois. La castration immunologique permettrait de stopper la sécrétion des hormones hypophysaires au moment le plus favorable pour la production de viande et sans utiliser de méthodes brutales.

L'invention concerne par conséquent des conjugués d'haptène et de muramyl-peptides, dans lesquels la partie haptène présente un poids moléculaire suffisamment faible pour annuler l'effet pyrogène qui est susceptible d'être observé à l'occasio du couplage des mêmes muramyl-peptides sur des antigènes de poids moléculaire plus élevé. La partie haptène doit en outre encore présenter un poids moléculaire suffisamment faible pour que le conjugué obtenu ne soit pas apte à induire en sus de l'immunogénicité attendue, la production d'anticorps dirigés contre la partie muramyl-peptide elle-même du conjugué.

En particulier, l'invention concerne des conjugués de muramyl-peptides et d'haptènes dont les poids moléculaires ne dépassent pas 5000 et avantageusement sont inférieurs à 3000. Dans des cas avantageux, les poids moléculaires des conjugués immunogènes et apyrogènes sont au plus de l'ordre de 2000. On peut observer que, sauf quelques exceptions notables (notamment LH-RH), les haptènes entrant dans la catégorie à laquelle l'invention s'intéresse sont, à l'état non conjugué, dépourvus d'immunogénicité décelable, et ce même en association avec du MDP.

Les haptènes auxquels l'invention s'intéresse peuvent encore être définis comme consistant en ceux qui ont un poids moléculaire suffisamment faible pour que les conjugués qu'ils sont susceptibles de former avec le MDP ne conduisent pas à une réaction pyrogène dépassant l'induction d'un accroissement de température de 0,6 °C, lorsqu'ils sont administrés par voie intraveineuse à des lapins, à raison de doses pouvant atteindre 100 microgrammes par kilo, voire 300 et même 500 microgrammes de conjugué par kilo.

S'agissant de peptides, synthétiques ou non, les haptènes mis en jeu dans l'invention comprennent de préférence moins de 50 résidus aminoacyle (à l'exclusion de l'eicosapeptide C-terminal de la β-hCCG), notamment moins de 30, de préférence moins de 20 aminoacyles, avantageusement moins de 15 aminoacyles. L'invention peut encore mettre en jeu des haptènes oligomérisés, pour autant que les oligomères répondent aux autres conditions énoncées ci-dessus, en ce qui concerne les haptènes susceptibles de la conjuguaison pour former des immunogènes.

A titre d'exemple d'haptènes susceptibles d'être utilisés pour la fabrication des conjugués selon l'invention, on mentionnera en premier lieu des hormones, notamment de la catégorie des hormones peptidiques ou glycopeptidiques. Parmi celles-ci, on mentionnera le facteur de libération de la lutéoptropine, connue sous la désignation LH-RH, des fragments peptidiques, ou des peptides synthétiques doués de propriétés analogues, par exemple des décapeptides du type

p-Glu-His-Trp-Ser-Tyr-Gly-Leu-Arg-Pro-Gly-$NH_2$

ou l'acide libre correspondant

p-Glu-His-Trp-Ser-Tyr-Gly-Leu-Arg-Pro-Gly.

On mentionnera en outre plus particulièrement le peptide C-terminal de la gonadotropine chorionique humaine (hCG), à l'exception de l'eicosapeptide. On mentionnera à titre d'exemple des peptides C-terminaux de la sous-unité β de la hCG, notamment un triacontapeptide contenant les résidus aminoacyles correspondant à des séquences contenues dans l'hCG-β ou de dérivés

synthétiques terminaux du genre de ceux décrits par S. MATSUURA et al. (publication déjà mentionnée plus haut), des fragments de FSH, etc., ou des peptides consistant essentiellement en les séquences 109–145 et 111–145 des sous-unités C-terminales de hCG-β décrites par Arthur C.J. LEE et al. dans «Molecular Immunology», vol. 17, p. 749–756. La modification de ces hormones par couplage avec des muramyl-peptides conduit à des principes immunogènes susceptibles d'induire in vivo la production d'anticorps contre les hormones naturelles en question, immunogénicité d'autant plus remarquable que les haptènes modifiés immunogènes ainsi obtenus ont des poids moléculaires extrêmement faibles.

On mentionnera encore les conjugués mettant en jeu le couplage entre un muramyl-peptide et différents peptides synthétiques ayant des séquences de résidus aminoacyles en commun avec les principaux peptides constitutifs de l'antigène HBsAg, tels qu'identifiés dans les publications déjà mentionnées, notamment ceux dont les aminoacyles d'extrémité correspondent à ceux qui, dans la séquence de HBsAg occupent les positions suivantes: 48–81; 2–16; 22–35; 95–109; 117–137; 122–137; 117–135 (selon la structure donnée par PASEK et al. et reproduite dans l'article de LERNER). On citera en outre des peptides synthétiques du genre de ceux décrits par E.H. BEACHEY et al. qui sont susceptibles, lorsque couplés avec un muramyl-peptide, de former des anticorps actifs contre la protéine M de la surface de Streptococcus piogenes. On peut également se référer aux séquences peptidiques définies dans le brevet américain No 4 284 537, plus particulièrement celles définies sous les abréviations $CB_6$ et $CB_7$.

A titre d'haptènes saccharidiques ou osidiques, on mentionnera par exemple les groupes sanguins A et B, le polysaccharide C du Streptococcus C, l'acide cellobiuronique (Pneumo Type III), tel que décrit par W.F. GOEBEL, «J. Exp. Medicine», 1939, p. 353–363.

On se reportera encore avec avantage, en ce qui concerne les différents types d'haptènes susceptibles d'être mis en jeu dans le couplage selon l'invention, aux séquences peptidiques décrites par Thomas P. HOPP et al. dans «Proc. Natl. Acad. Sci. USA», vol. 78, no 6, p. 3824–3828, dans lesquelles sont passées en revue d'autres séquences peptidiques susceptibles d'être rendues antigéniques par couplage avec des macromolécules supports, mais qui, dans le cadre de la présente invention, peuvent également être rendues immunogènes par couplage à un dérivé de muramyl-peptide.

Bien entendu, les différents peptides et autres types d'haptènes visés dans la littérature et qui, à ce titre, peuvent être utilisés pour constituer des agents immunogènes tels que définis dans la présente demande de brevet, ne constituent que des exemples des nombreux haptènes qui peuvent être mis en œuvre. Il va de soi que des haptènes répondant aux conditions de l'invention et correspondant à un antigène déterminé ne sont pas toujours immédiatement disponibles. Lorsque la constitution de l'antigène est ou peut être connue, notamment lorsque celui-ci consiste en un polypeptide dont la séquence est connue ou peut être déterminée, il appartiendra au spécialiste de rechercher ceux des éléments de la structure de cet antigène qui seraient susceptibles de porter un site immunogénique dans les conditions qui ont été indiquées ci-dessus. Plusieurs approches à l'égard de cette question ont déjà fait l'objet de publications antérieures. Pour mémoire, on citera par exemple les articles de LERNER et HOPP susmentionnés, dans lesquels sont proposées des méthodes d'investigation permettant un repérage des séquences contenues dans un antigène de structure peptidique et susceptible de porter un site immunogénique, les séquences en question pouvant ensuite être synthétisées et testées de façon particulièrement commode lorsque l'on a recours à la technique de l'invention, c'est-à-dire par couplage avec un muramyl-peptide, pour leur capacité immunogène et plus particulièrement leur capacité d'induire la production in vivo d'anticorps actifs contre l'antigène natif.

Les haptènes peuvent dans les conjugués selon l'invention être couplés à tous dérivés de muramyl-peptide présentant des propriétés d'adjuvants immunologiques.

La partie muramyl-peptide conjugée à l'haptène est avantageusement dérivée du muramyl-peptide qui répond à la formule générale (I)

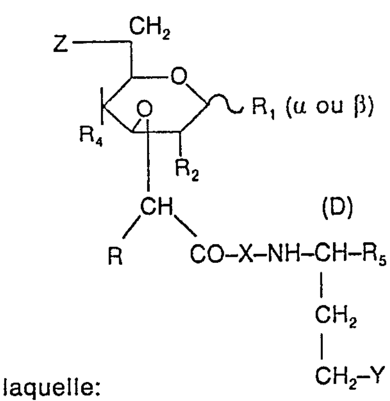

(I)

dans laquelle:

– $R_5$ est une fonction carboxyle libre ou une fonction amidée, ou une fonction carboxyle estérifiée par un alcool aliphatique possédant de 1 à 10 atomes de carbone, ou une fonction carboxyle substituée par un acide aminé possédant à son tour une fonction carboxyle libre, amidée ou estérifiée comme sus-indiqué,

– R est un hydrogène ou un méthyle,

– R2 est un groupe actétamido ou glycolylamido,

– $R_1$ est soit $-NH_2$, soit OH, soit un radical comprenant au plus 10 atomes de carbone, correspondant à un groupe alkyle, aryle, arylalkyle ou alkylaryle, portant le cas échéant des substituants hydroxyle, amine, carboxamide, sulfonamide, éther, thioéther, ester ou tioester.

– $R_4$ est un hydroxyle ou un groupe oxyacyle comprenant de 1 à 4 atomes de carbone, notamment un groupe oxyacètyle,

– X est un résidu aminoacyle du groupe comprenant:

alanyle, arginyle, asparagyle, aspartyle, cystéinyle, glutaminyle, glutamyle, glycyle, histidyle, hydroxyprolyle, isoleucyle, leucyle, lysyle, méthionyle, phénylalanyle, prolyle, séryle, thréonyle, tryptophanyle, tyrosyle, valyle et aminobutyryle,

– Z est un groupement substituant la position en C-6 de l'acide muramique, qui répond à la formule générale (II) suivante:

$$-R_6-(R_7)_x-H \quad \text{(II)}$$

dans laquelle x = 0 ou 1 et dans laquelle
si x = 0,
– $R_6$–H est un groupe hydroxyle ou amine et
si x = 1,
– $R_6$ est une liaison oxygène ou NH, carboxamide, sulfonamide, éther ou thioéther, ester ou thioester,
– $R_7$ étant alors un enchaînement de groupes $-CH_2-$, $-CH=CH-$, $-S-$, $-CO-$ ou $-NH-$, les groupes $-CH_2-$; $-CH=CH-$, ou $-NH-$ pouvant éventuellement être remplacés ou substitués par des groupes aryle, de préférence phényle, ou hétérocyclique comme ci-dessous définis, ou encore être substitués par des fonctions carboxylique, amino, sulfhydryle ou hydroxyle, ou par des groupes alkyle susceptibles de comporter jusqu'à 5 atomes de carbone ou des groupes alkylaryle, arylalkyle comportant en tout jusqu'à 8 atomes de carbone ou par des groupes hétérocycliques comportant jusqu'à six atomes de carbone et un ou deux atomes d'oxygène, d'azote ou de soufre, les groupes alkylaryle ou arylalkyle susdits pouvant contenir des groupes carbonyle ou encore être substitués par des groupes carboxyle, amino, sulfhydryle ou hydroxyle et les susdits groupes hétérocycliques pouvant eux-mêmes être substitués par des fonctions alkyle pouvant contenir jusqu'à 5 atomes de carbone, carboxyle, amino, sufhydryle ou hydroxyle, le susdit groupe $R_7$ ayant néanmoins une longueur ne dépassant pas celle d'une chaîne hydrocarbonée n-décylique,
– Y est une fonction carboxyle libre ou amidée, ou ester comprenant jusqu'à 10 atomes de carbone, ou substituée par un acide aminé, de préférence un acide aminé trifonctionnel, c'est-à-dire comportant, en sus d'une fonction carboxyle et d'une fonction amine, une fonction supplémentaire carboxyle, amine ou hydroxyle, notamment la lysine, l'ornithine, l'acide glutamique, l'acide aspartique ou la tyrosine; ou Y est un groupement qui répond à la formule générale III:

$$-R_8-(R_9)_y-H \quad \text{(III)}$$

dans laquelle y est égal à zéro ou à 1 et dans laquelle $R_8$–H est un groupe amide ou ester, lorsque y est égal à zéro, ou lorsque y est différent de zéro, $R_8$ est une liaison carboxamide, ester ou thioester, $R_9$ ayant alors la même signification que $R_7$.

Une classe préférée de muramyl-peptides répondant à la susdite formule générale consiste en ceux dans lesquels X est un résidu glycyle, séryle, valyle, leucyle, aminobutyryle, ou de façon encore davantage préférée alanyle.

Tous ces aminoacyles sont de préférence (à l'exception de la glycine) des aminoacyles lévogyres.

Dans chacune des classes qui précèdent, des sous-classes préférées répondent à la formule susdite, cependant avec x étant égal à zéro, cas dans lequel Z est avantageusement une fonction hydroxyle ou amine.

Dans chacune des classes précédemment définies, une autre sous-classe préférée répond à la formule (I) sus-indiquée, dans laquelle:
– x = 1,
– $R_6$ est une liaison carboxamide ou sulfonamide éther ou thioéther, ester ou thioester, et
– $R_7$ représente l'un des groupements tels que ceux indiqués ci-dessous:
– $(CH_2)_n-COOH$, notamment avec n = 1, 2, 3 ou 4
– $(CH_2)_n-NH_2$; notamment avec n = 1, 2, 3 ou 4

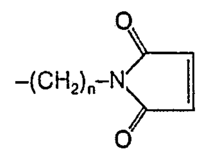

notamment avec n = 1, 2, 3 ou 4
– $(CH_2)_n-C_6H_5-NH_2$, notamment avec n = 0 ou 1
– $(CH_2)_n-S\,H$, notamment avec n = 2

L'invention concerne encore les conjugués dérivés de muramyl-peptides appartenant à l'une quelconque des classes et sous-classes sus-indiquées dans lesquels le groupe $R_5$ est une fonction carboxyle estérifiée par l'alcool n-butylique, et de préférence encore ceux dans lesquels simultanément Y est un groupe $-CONH_2$.

L'invention concerne encore, à l'intérieur de chacune des classes et sous-classes sus-indiquées, les catégories préférées de conjugués haptène-muramyl-peptides, dans lesquelles le groupe R du groupement muramyl-peptide est un groupe méthyle.

L'invention concerne encore, à l'intérieur de chacune des classes, sous-classes et catégories sus-indiquées, celles, préférées, dans lesquelles le groupe $R_5$ de la partie muramyl-peptide du conjugué selon l'invention est un groupe ester comportant de 1 à 5 atomes de carbone.

L'invention concerne encore, à l'intérieur des classes, sous-classes et catégories sus-indiquées, une sous-catégorie de conjugués dans lesquels le groupe Z de la partie muramyl-peptide est un groupe succinyle.

L'invention concerne encore, à l'intérieur de chacune des classes, sous-classes, catégories et sous-catégories sus-indiquées, une autre sous-catégorie de conjugués dans lesquels la partie muramyl-peptide des conjugués concernés, $R_1$ et $R_4$ sont sumultanément des hydroxyles et $R_2$ un groupe acétamido.

D'autres sous-catégories préférées de conjugués selon l'invention, respectivement dans les susdites classes, sous-classes et catégories, contiennent une partie muramyl-peptide, dans laquelle simultanément Z, $R_1$ et $R_4$ sont des hydroxyles, $R_2$ est un groupe actétamido, $R_4$ est un groupe méthyle, X est un groupe alanyle, $R_5$ est un groupe carboxamide ou alternativement ester comportant de 1 à 5 atomes de carbone, et Y a une fonction carboxamide.

Des conjugués encore préférés selon l'invention sont ceux qui contiennent une partie muramyl-peptides dérivés du MDP, de l'α-n-butyl-ester de la N-acétyl-muramyl-L-alanyl-D-glutamine ou encore des dérivés 6-0-succinylés des deux muramyl-peptides précédents.

D'autres muramyl-peptides préférés entrant dans la composition des conjugués selon l'invention, sont des mono-α- ou γ-esters ou des diesters de l'acide N-acétyl-muramyl-L-alanyl-D-glutamique (MDPA), les groupes ester contenant de 1 à 5 atomes de carbone.

D'autres catégories de conjugués préférés selon l'invention sont ceux dérivés de muramyl-peptides dans lesquels Y est un groupe lysyle, les autres groupes portés par lesdits muramyl-peptides étant alors conformes aux définitions qui en ont été données en rapport avec chacune des catégories et sous-catégories sus-indiquées. Des conjugués préférés mettent en jeu la N-acétyl-muramyl-L-alanyl-D-isoglutaminyl-L-lysine (MTP).

L'invention concerne aussi un procédé pour fabriquer ces conjugués entre un haptène et un muramyl-peptide appartenant à l'une des classes, sous-classes ou catégories sus-indiquées, ce procédé comportant diverses variantes possibles.

Le procédé selon l'invention est caractérisé par une réaction de condensation entre les deux partenaires que constituent l'haptène et le muramyl-peptide, l'un de ces partenaires étant pourvu d'une fonction aminée carboxyle, ester, alcool, sulfhydryle ou amine, et l'autre de ces partenaires étant pourvu d'une fonction complémentaire, la réaction étant conduite en présence d'un agent de condensation, et en ce que l'haptène présente un poids moléculaire ne dépassant pas 5000, et qui de préférence est inférieur à 3000. De préférence encore, l'haptène et le muramyl-peptide respectivement mis en œuvre sont choisis de façon que le poids moléculaire du conjugué immunogène et apyrogène obtenu soit au plus de l'ordre de 2000. On peut observer que, sauf quelques exceptions notables (notamment LH-RH), les haptènes entrant dans la catégorie à laquelle l'invention s'intéresse sont, à l'état non conjugué, dépourvus d'immunogénicité décelable, et ce même en association avec du MDP.

Les haptènes préférés mis en œuvre dans le procédé selon l'invention ont un poids moléculaire suffisamment faible pour que les conjugués qu'ils sont susceptibles de former avec le MDP ne conduisent pas à une réaction pyrogène. De préférence, ces haptènes sont choisis parmi ceux qui conduisent à des conjugués n'induisant pas un accroissement de température de 0,6 °C, lorsqu'ils sont administrés par voie intraveineuse à des lapins, à raison de doses pouvant atteindre 100 microgrammes par kilo, voire 300 et même 500 microgrammes de conjugué par kilo.

Lorsque les haptènes choisis sont des peptides, synthétiques ou non, ils comprennent de préférence moins de 50 résidus aminoacyle (à l'exclusion de l'eicosapeptide C-terminal de la β-hCG), notamment moins de 30, de préférence moins de 20 aminacyles, avantageusement moins de 15 aminoacyles. L'invention peut encore mettre en jeu des haptènes oligomérisés, pour autant que les oligomères répondent aux autres conditions énoncées ci-dessus, en ce qui concerne les haptènes susceptibles de la conjuguaison pour former des immunogènes.

Des exemples d'haptènes susceptibles d'être utilisés pour la fabrication des conjugués selon l'invention, ont été indiqués plus haut, en rapport avec la description de conjugués préférés selon l'invention.

Les haptènes peuvent dans le procédé selon l'invention être couplés à tous dérivés de muramyl-peptide présentant des propriétés d'adjuvants immunologiques, plus particulièrement un muramyl-peptide comprenant au moins une fonction carboxyle, ester, alcool, sulfhydryle ou amine, apte à intervenir dans le couplage covalent avec une fonction complémentaire correspondante de l'haptène, ce muramyl-peptide répondant à la formule générale (I) susdite.

Les muramyl-peptides préférés, qui sont mis en œuvre dans le procédé selon l'invention appartiennent aux classes, sous-classes, catégories et sous-catégories préférées qui ont été définies plus haut, en ce qui concerne les groupes muramyl-peptide entrant dans la constitution des conjugués préférés de l'invention.

Il va de soi que le muramyl-peptide choisi pour réaliser la réaction de couplage selon l'invention devra toujours comprendre au moins une fonction amine, carboxyle libre, ester ou alcool susceptible d'être mise en jeu dans la réaction de couplage avec la fonction correspondante portée par l'haptène. Si besoin, les fonctions réactives portées par le muramyl-peptide, autres que la fonction devant intervenir dans la réalisation d'un couplage covalent entre le muramyl-peptide et l'haptène, pourront être protégées s'il en est besoin.

De préférence, la réaction de couplage met en jeu des positions déterminées du muramyl-peptide. En particulier, ces liaisons pourront intervenir au niveau soit de la position en $C_6$, soit de celle en $C_1$ du cycle saccharidique, soit encore à l'extrémité C-terminale Y de la chaîne peptidique.

Conformément à une première variante du procédé selon l'invention, on réalise la condensation entre un haptène portant au moins une fonction amine libre et un muramyl-peptide portant également au moins une fonction amine libre et l'on procède à la condensation de ce muramyl-peptide et de cet haptène, en présence d'un réactif difonctionnel tel que, par exemple, la glutaraldéhyde ou la benzoquinone.

Selon une autre variante du procédé de l'invention, on fait réagir l'haptène portant une fonction réactive amine, carboxyle, hydroxyle ou sulfhydryle selon l'une des techniques couramment utilisées en synthèse peptidique, avec le muramyl-peptide qui porte alors lui-même une fonction terminale complémentaire amine, carboxyle, hydroxyle, alcool ou sulfhydryle, soit au niveau du groupe Z, soit au niveau du groupe Y, selon que l'on souhaite réaliser la fixation au niveau du carbone en 6 du groupe saccharidique, ou au contraire au niveau de la fonction γ-carboxyle du muramyl-peptide.

Lorsque l'on réalise le couplage entre une fonction carboxyle portée par l'un des partenaires (haptène ou muramyl-peptide) avec une fonction amine portée par l'autre partenaire de la réaction (muramyl-peptide ou haptène), on réalise avantageusement la réaction en phase aqueuse en présence d'un carbodiimide hydrosoluble ou d'un carbodiimide liposoluble au sein d'un solvant organique inerte, tel que le diméthyl-formamide (DMF) ou la tétrahydrofuranne (THF), acétate d'éthyle ou chlorure de méthylène.

Des carbodiimides hydrosolubles avantageux sont constitués par le N-cyclohexyl-N'-(β-(N-méthyl-morpholino)-éthyl)-carbodiimide p-toluène sulfonate, le chlorhydrate de (3-méthyl)-aminopropyl-carbodiimide.

Des carbodiimides appropriés pour des réactions au sein d'un solvant organique sont par exemple constitués par le N,N'-dicyclohexyl-carbodiimide.

Selon une autre variante du procédé selon l'invention, on fait réagir la fonction carboxyle portée par l'un des partenaires avec un chlorocarbonate d'alkyle ($C_2$ à $C_4$), puis l'on fait agir l'anhydride mixte obtenu à partir de ce premier partenaire avec le second partenaire portant la fonction amine devant intervenir dans le couplage au sein d'un solvant organique inerte, tel que ci-dessus défini, ou en phase aqueuse.

La première étape de la réaction est effectuée par exemple dans le diméthylformamide, en présence d'une amine tertiaire, par exemple la N-méthyl-morpholine, en utilisant par exemple le chlorocarbonate d'isobutyle. Après trois minutes à $-15\,°C$, on ajoute au mélange réactionnel le partenaire porteur d'une fonction amine déprotonée, par exemple avec de la N-méthyl-morpholine.

Selon une troisième variante du procédé selon l'invention, on fait réagir un ester activé préalablement formé de celui des partenaires portant le groupe carboxylique devant intervenir dans la réaction de couplage, avec l'autre partenaire portant une fonction amine. Des esters activés avantageux sont l'ester p-nitrophénylique ou l'ester N-hydroxysuccinimidique.

Ces réactions sont conduites dans un solvant organique inerte, tel que le diméthyl-formamide.

Selon une autre variante encore du procédé selon l'invention, on fait réagir l'un des partenaires portant une fonction sulfhydryle avec l'autre partenaire, lequel porte alors un groupe maléimide.

On peut encore faire réagir avec le partenaire portant le groupe sulfhydryle l'autre partenaire portant un groupe 6-maléimido-caproïque-acide ou un groupe 3-(2-pyridyl-dithio)propionate.

Les fonctions réactives ne devant pas intervenir dans la réaction peuvent être protégées par des groupes de blocage classiques.

Les groupes carboxyliques peuvent notamment être protégés par des groupes benzylique, benzylidène ou anzilidène.

Ces groupes esters peuvent ensuite être éliminés notamment par hydrogénolyse. Celle-ci permet d'ailleurs également l'élimination simultanée d'autres groupes protecteurs tels que les groupes N-carbobenzoxy.

Les fonctions amine sont avantageusement protégées par des groupes benzyl-oxycarbonyle, t-butyl-oxycarbonyle ou toluène sulfonyle. Ces groupes peuvent ensuite être éliminés par hydrogénation catalytique ménagée, par exemple en présence de palladium, ou par acidolyse ou encore par action du sodium dans l'ammoniac liquide.

Les fonctions sulfhydryle n'intervenant pas dans la réaction de couplage peuvent être protégées par des groupes acétamidométhyle ou formamidométhyle.

On peut aussi encore se reporter aux techniques générales envisagées dans la demande de brevet européen EP-A-3 833.

Bien entendu, on peut avoir recours à tout autre type de liaison, mettant en jeu les combinaisons pouvant intervenir entre une fonction sulfhydryle portée par l'un des partenaires que constituent l'haptène et le muramyl-peptide et un groupe maléimide porté par l'autre réactif, par exemple en faisant usage de la technique décrite par T. KITAGAWA et T. AIKAGAWA dans «J. Biochem.» 79 (1976), 233.

La conjuguaison peut également se faire en utilisant les modes classiques de diazotisation ou de réaction mettant en jeu un isothiocyanate, notamment lorsque le muramyl-peptide porte une fonction aromatique aminée (notamment au niveau des groupes Z ou $R_1$), et lorsque l'haptène porte une fonction amine susceptible d'intervenir dans cette réaction. De telles techniques de réalisation sont décrites par exemple par B.F. ERLANGER dans «Pharmacol. Rev.», 25 (1973), 271.

Le couplage peut également être réalisé par la réduction d'une base de Schiff réalisée entre une fonction aldéhyde portée par l'un des réactifs et une fonction amine portée par l'autre (G.R. GRAY, «Arch. Biochem. Biophys.», 163 (1974), 421).

Toutes ces réactions sont en soi bien connues et le spécialiste appréciera que de nombreuses variantes peuvent être aménagées pour aboutir aux mêmes résultats. En ce qui concerne d'ailleurs plus particulièrement les groupes Y et Z, on remarquera aussi que, entre le muramyl-peptide et l'haptène, les conjuguaisons peuvent s'effectuer au moyen d'un pontage, l'agent de pontage consistant par exemple en un réactif bifonctionnel.

En l'occurrence, le groupe de pontage entre le muramyl-peptide et l'haptène dans le conjugué final de préférence n'excèdera pas la longueur d'un enchaînement correspondant à celle d'une chaîne hydrocarbonée n-décylique.

De tels agents de pontage sont par exemple mentionnés dans le brevet FR-A-2 428 050.

Dans ce qui précède, il a surtout été évoqué le cas du couplage entre un muramyl-peptide et un haptène de nature peptidique. Lorsque l'haptène est constitué par un oligosaccharide, les mêmes types de réactions peuvent être mis en œuvre, dès lors que l'haptène possède une fonction carboxylique libre ou une fonction amine, ou encore une fonction alcool pouvant alors former des liaisons amide ou ester avec des fonctions adéquates portées par le muramyl-peptide.

En variante, on pourra également avoir recours à une fixation du muramyl-peptide sur la position en $C_1$ de l'oligosaccharide, possédant un sucre terminal réducteur, soit que la fonction pseudoaldéhyde soit oxydée en une fonction carboxylique (G. ASHWELL, «Methods in Enzymol.» 28 (1972), 219), soit qu'elle soit utilisée en tant que telle (R. GRAY déjà cité). Dans le premier cas, on établit une liaison peptidique ou ester; dans le second cas une liaison alkylamine.

La fixation de l'haptène sur le muramyl-peptide peut intervenir en plusieurs points de celui-ci, l'inverse étant également vrai, de sorte que l'invention s'étend à des oligomères de muramyl-peptide et de l'haptène, dans la mesure où les deux constituants présentent les fonctions chimiques appropriées, étant entendu cependant que le poids moléculaire de tels conjugués ne doit normalement pas dépasser 5000.

D'une façon générale, le constituant haptène et le constituant muramyl-peptide seront avantageusement dans un rapport pondéral compris entre 1,5 et 10, avantageusement entre 2 et 3.

L'invention concerne naturellement également les compositions préparées sous forme de vaccins contenant le conjugué selon l'invention, en association avec un véhicule approprié.

Des compositions pharmaceutiques avantageuses sont constituées par des solutions, suspensions ou liposomes injectables, contenant une dose efficace d'au moins un conjugué selon l'invention. De préférence, ces solutions, suspensions ou forme liposome sont réalisées dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autres voies, notamment par voie orale ou rectale, ou encore sous forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des conjugués selon l'invention se trouve associé à des excipients pharmaceutiquement acceptables, solides ou liquides, adaptés à la constitution de formes orale, oculaire ou nasale, ou avec des excipients adaptés à la constitution de formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaire ou nasale. Elle concerne enfin des compositions formées de gaz liquéfiés pharmaceutiquement acceptables, dans lesquels les conjugués selon l'invention sont dissous ou maintenus en suspension, et dont la détente provoque la dispersion en un aérosol.

Avantageusement, les compositions vaccinales selon l'invention contiennent en outre un véhicule, tel que la polyvinyl-pyrrolidone, facilitant la démonstration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthylcellulose, les hydroxydes et phosphates d'aluminium ou tous autres adjuvants de ce type, bien connus de l'homme de l'art.

Les compositions pharmaceutiques selon l'invention sont donc essentiellement destinées à induire chez l'hôte une réaction immunitaire, lui permettant de produire des anticorps ou une immunité à médiation cellaire à l'égard soit de l'antigène ayant une séquence peptide en commun avec l'haptène, soit de l'haptène lui-même. Ce dernier cas est d'un intérêt particulier lorsque l'haptène est constitué d'un peptide naturel, tel qu'une hormone, dont on veut pallier les effets. Ces derniers types de compositions pharmaceutiques seront dont particulièrement utiles en thérapeutique humaine ou vétérinaire, chaque fois que sera recherchée une modification de l'évolution ou du comportement de l'organisme humain ou animal, par exemple au niveau du contrôle de la fécondité d'une espèce animale ou de l'orientation dirigée du métabolisme de l'organisme de l'hôte humain ou animal vers la suppression de certaines hormones. Dans ce dernier cas, l'haptène entrant dans le conjugué mis en œuvre pourra être constitué par l'hormone elle-même ou un fragment de celle-ci.

Dans le domaine vétérinaire, on citera en outre la production accrue de certains constituants, au détriment d'autres constituants. On citera par exemple les applications des vaccins vétérinaires administrés dans le but d'accroître chez l'animal la production de viande par castration immunologique.

L'invention a en particulier pour objet des conjugués d'haptène, dans lesquels l'haptène lui-même est constitué soit par l'hormone elle-même, soit par une séquence peptidique ayant une partie commune avec l'hormone, l'hormone appartenant à la classe des hormones peptidiques ou glycopeptidiques. Une hormone particulièrement intéressante à cet égard est constituée par le facteur de libération de l'hormone lutéostimulante LH-RH.

L'invention concerne également encore des réactifs biologiques essentiellement formés à partir de ces conjugués haptène-anticorps. En particulier, la partie hapténique pourra être constituée par un principe actif de médicament ou de drogue (par exemple la morphine), le réactif biologique en question étant alors utilisé pour fabriquer des anticorps d'apyrogénicité réduite, notamment d'anticorps monovalents, éventuellement même monoclonaux. Ces anticorps seront alors à leur tour utiles pour la constitution de réactifs permettant par exemple l'étude de l'action de médicaments en cause sur l'hôte vivant. En particulier, ces anticorps permettront la détection de récepteurs cellulaires, ou encore l'étude du métabolisme et du mode d'action des médicaments en cause.

L'invention concerne encore, et ce d'une façon générale, les anticorps eux-mêmes ou la préparation d'anticorps tels qu'ils sont obtenus par administration à un hôte vivant des conjugués d'haptène et de muramyl-peptides selon l'invention. Une caractéristique d'identification essentielle de ces anticorps consiste naturellement dans la capacité qu'ils possèdent de neutraliser les conjugués selon l'invention et, en outre, lorsque la partie hapténique de ces derniers possède un élément de structure commun avec un antigène natif, également ce dernier.

L'invention fournit, en particulier, comme cela est illustré ci-après dans l'un des exemples un conjugué de cette dernière hormone avec un muramyl-peptide ayant un poids moléculaire inférieur à 2000 daltons, présentant une immunogénicité élevée et en même temps un effet pyrogène négligeable, voire inexistant, même lorsque le muramyl-peptide entrant dans le conjugué présente lui-même un certain niveau de pyrogénicité. En outre, et contrairement à ce qui s'observe avec les conjugués de l'état de la technique formés contre les antigènes de poids moléculaire plus élevé et les muramyl-peptides, ceux de l'invention sont dépourvus d'action immunogène à l'égard du constituant muramyl-peptide, en tous les cas dans les limites de ce qui peut être détecté avec les méthodes de détection en usage à ce jour.

D'autres caractéristiques de l'invention apparaîtront encore au cours de la description qui suit d'exemples préférés de mise en œuvre de l'invention, ainsi que des résultats que les conjugués selon l'invention permettent d'obtenir.

Synthèse de (LH-RH)-MTP

Pour obtenir une réaction de conjugaison typique, on ajoute 3 mg de LH-RH sous forme acide à 242 mg de chlorhydrate de N-éthyl-N'-(3 méthyl-aminopropyl)-carbodiimide (EDCI) et à 43 mg de 1-hydroxy-benzotriazole (HOBT), au sein de 0,4 ml de diméthylformamide (DMF) agissant comme solvant. L'incubation est effectuée sous agitation magnétique pendant 7 heures à température ambiante. Puis on ajoute à cette solution 3,24 mg de MTP (N-acétyl-muramyl-L-alanine-D-isoglutaminyl-L-Lysine) mélangés à 0,3 ml d'eau distillée et on laisse la réaction se poursuivre sous agitation magnétique pendant 48 heures à température ambiante. On reprend les produits de la réaction dans une solution de NaCl 0,15 M et on récupère les conjugués formés par fractionnement sur tamis moléculaire de SEPHADEX® G10. Les conjugués sont élués dans le volume mort. Les teneurs en peptides et en acide muramique sont évalués selon la méthode de Reissig et al., J. Biol. Chem. 217, 959–966 (1956). On trouve que 93% du MTP sont couplés: ainsi le nombre de molécules MTP par molécule de LH-RH approche 1:1.

Synthèse du conjugué MDP-toxine tétanique (fabriqué à titre de témoin)

On ajoute à 16 mg de MTP, 0,15 ml de DMF plus 0,01 ml de PBS à pH 7,4 (solution saline tamponnée au phosphate) puis 300 µg de ECDI, 1 mg de HOBT et 2 mg de solution de toxine tétanique. On laisse la réaction se poursuivre pendant 15 minutes sous agitation magnétique, à température ambiante et dans l'obscurité; puis on ajoute à cette mixture 300 µg de EDCI, 1 mg de HOBT et 2 mg de toxine tétanique; la réaction se poursuit encore pendant 15 minutes et la même opération est répétée 9 fois. On constate alors que 20 mg de toxine tétanique ont été utilisés. A la fin de la réaction on récupère le conjugué par chromatographie sur colonne SEPHADEX G75 dans PBS. Le conjugué est contenu dans le premier pic d'élution. Les dosages respectifs de toxines tétanique et de MTP sont effectués par la méthode de Folin et Reissig.

Couplage de MTP à des érythrocytes frais

On effectue le couplage du MTP à des érythrocytes frais par l'intermédiaire de la benzoquinone, en mettant en œuvre le procédé suivant: on ajoute 0,2 ml de benzoquinone (30 mg/ml) à 3 mg de MTP dissous dans 0,2 ml de PBS 0,2 M à pH 7. On laisse la réaction se poursuivre pendant 90 minutes à température ambiante, dans l'obscurité, sous agitation. La réaction est arrêtée par chromatographie sur une colonne de tamis moléculaire SEPHADEX G25 en milieu de $NaHCO_3$, 0,1 M, pH 0,8. Le MTP activé par la benzoquinone, est élué dans le volume mort. On l'ajoute à 0,8 ml d'une suspension de 50% de globules rouges frais de mouton dans une solution de $NaHCO_3$ 0,1 M. On laisse incuber le mélange pendant la nuit à température ambiante sous agitation, puis on lave trois fois les globules retenant le MTP avec du PBS.

Immunisation par LH-RH couplé au muramyl-peptide et administré dans une solution saline ou un adjuvant de Freund incomplet (FIA)

On administre par injection sous-cutanée et aux jours 0, 2, 4 et 29 un volume de 0,1 ml de la solution suivante à plusieurs groupes de souris Swiss mâles, âgées de 6 semaines

1) 50 µg de LH-RH émulsifié dans de l'adjuvant complet de Freund

2) 50 µg de LH-RH émulsifié dans du FIA

3) 8,6 µg de LH-RH conjugué avec 4,3 µg de MDP et émulsifié dans du FIA

4) 50 µg de LH-RH dans du PBS

5) 8,6 µg de LH-RH conjugué avec 4,3 µg de MDP dans du PBS.

On injecte à trois groupes témoins supplémentaires, soit du FCA, soit du FIA, soit du PBS, sans antigène ni MDP.

Il faut noter qu'avant usage, l'antigène (LH-RH pur ou couplé) a été adsorbé sur 50% de PVP (polyvinylpyrrolidone) dans 0,9% de NaCl et que l'incubation s'est poursuivie pendant 2 heures; puis les mélanges ont été émulsifiés avec FIA ou FCA ou ajoutés au PBS.

Le volume de 0,1 ml injecté aux souris par voie sous-cutanée contenait 12,5% de PVP.

Aux jours 23 et 50, toutes les souris sont saignées, sacrifiées et leurs sérums recueillis et réunis pour chacun des groupes. Leurs testicules et vésicules séminales sont prélevés, pesés, mis dans une solution de Boin et préparés pour l'étude histologique.

Détection d'anticorps anti-(LH-RH)

On détecte les anticorps anti-(LH-RH) comme suit:

On ajoute 50 µl du sérum non dilué devant être testé, 50 µl de sérum normal de lapin dilué au $^{1}/_{80ème}$ dans le PBS et 10 µl de LH-RH marqué à l'iode 125 (approximativement 10 000 désintégrations par minute:dpm). On laisse l'incubation se poursuivre pendant 72 heures à 4 °C. Puis on ajoute 150 µl d'une suspension refroidie dans la glace, de charbon actif revêtu de dextrane (obtenue en ajoutant 250 mg de charbon activé à 100 ml d'une solution de dextrane dans une solution 0,01 M de PBS). On laisse à 4 °C pendant 5 minutes. Après centrifugation à 2500 tours/minute pendant 20 minutes, le surnageant est décanté et sa radioactivité est déterminée, selon la méthode de HABER et al., «Manual of Clinical Immunology» édité par Rose N.R. & Friedman H. pp. 190–196. Une étude préliminaire a indiqué que le charbon revêtu de dextrane pouvait absorber jusqu'à 95% de LH-RH pur dans les conditions utilisées.

Détection d'anticorps anti-MTP

On contrôle la présence d'anticorps anti-MTP au moyen d'un test hémolytique réalisé dans des plaques de microdosage pourvues de cavités en U (Cooke Engineering) de la façon suivante: les sérums sont décomplémentés par inactivation pendant 30 minutes à 56 °C. 50 µl de sérum ayant subi une double dilution sont mélangés avec 50 µl de PBS. On y ajoute 20 µg de conjugués frais globules rouges-MTP à une concentration de 2,5% et on laisse incuber les plaques à 37 °C pendant 15 minutes; finalement on ajoute 20 µl du complément (sérum normal de cobaye dilué à 1:20 avec PBS) et on laisse à nouveau incuber les plaques à 37 °C pendant 30 minutes, avant de noter les résultats.

Evaluation de la pyrogénicité

Les essais sont effectués sur des lapins de Nouvelle-Zélande de 2,5 à 2,8 kg. Les températures sont prélevées avec une sonde à thermistor introduite dans le rectum des lapins sur une longueur d'environ 8 cm et reliée à un téléthermomètre (modèle no TE3; Ellab, Copenhague, Danemark). Les lapins sont maintenus et observés dans une pièce climatisée à 20 °C. On ne pratique des injections que sur ceux dont la température est restée stable au cours des 30 minutes précédentes. Les températures sont mesurées pendant 3 heures après l'injection de la composition dont l'éventuelle pyrogénicité est étudiée. Les résultats sont exprimés par la variation moyenne de températures (°C) obtenue pour les lapins au cours des 3 heures qui suivent l'injection intraveineuse de la composition étudiée. Une dose pyrogène minimale correspond à la quantité produisant une élévation de 0,6 °C.

Les résultats suivants ont été constatés.

a) Essais d'immunisation.

Les résultats sont indiqués dans le tableau I ci-après. Ils sont exprimés comme suit: rapport du nombre de coups par minute (cpm) du surnageant au nombre de cpm total, et multiplié par 100.

TABLEAU I

| Traitement | Fixation (en %) |
|---|---|
| LH–RH 50 µg + FCA | 30,5 |
| LH–RH 50 µg + FIA | 2,5 |
| LH–RH 50 µg + PBS | 10 |
| (LH–RH)-MTP + PBS | 63 |

En l'absence d'antigène on n'observe aucune fixation dans les sérums des témoins injectés avec FCA, FIA ou PBS (la fixation mesurée (au maximum de 2,3%) n'étant pas significative). Des anticorps sont toutefois produits quand 50 µg de (LH-RH) sont émulsifiés dans FCA (30,5% de fixation). On ne détecte aucune réponse significative dans le groupe qui reçoit 50 µg de (LH-RH) dans FIA (fixation 2,5%) et la faible réponse obtenue avec PBS (10%) est sans signification. Une forte réponse est obtenue avec le sérum provenant d'animaux immunisés avec le conjugué MTP-(LH-HR) bien que le conjugué ne contienne que 8,6 µg de LH-RH et 4,4 µg de MTP. De façon étonnante, les réponses d'anticorps sont même supérieures lorsque des quantités égales de conjugué MTP-(LH-RH) ont été administrées dans un milieu aqueux. Ces résultats indiquent que les conjugués MTP-(LH-RH), sont tous deux plus efficaces que l'association de LH-RH et de FCA, tant dans le FIA que dans le PBS.

Etude histologique

Le poids moyen des testicules a légèrement augmenté (de 252 mg à 279 mg) par suite de l'administration de 5 ou 50 µg de LH-RH dans PBS. Bien que cette augmentation soit significative (p 0,05) elle n'est accompagnée par aucun changement histologique. Au contraire, la même quantité d'antigène administrée dans FCA produit une augmentation substantielle du poids des testicules, avec une diminution de la production de sperme et une légère atrophie des tubes séminifères dans certains cas. Au contraire chez les animaux ayant reçu le conjugué MTP-(LH-RH) en solution dans PBS, le poids des testicules est nettement plus bas (p 0,01) que dans les autres groupes. De plus, les études histologiques des testicules ont démontré seulement dans ce groupe une atrophie marquée à la fois des cellules interstitielles et des tubules séminifères, lesquels étaient pratiquement dépourvus de cellules germinatives dans 9 souris sur 10.

Mesure des anticorps anti-MTP.

Les mêmes sérums réunis, préalablement testés pour l'activité de fixation $^{125}$I-LH-RH ont également été étudiés pour ce qui est de leur contenu en anticorps anti-MTP au moyen d'un test hémolytique avec les conjugués de globules rouges et de MTP. Un sérum de lapin anti-MTP, obtenu après hyperimmunisation avec un conjugué d'alpha-méthylester de MTP et de sérum albumine bovine, est utilisé à titre d'antigène témoin. Alors que l'on constate des réactions d'hémolyse avec un sérum d'anti-MTP de lapin jusqu'à dilution finale de 1:30 · 720, on ne détecte aucun anticorps anti-MTP dans aucun des groupes traités avec le conjugué MTP-(LH-RH).

Détection de l'éventuelle pyrogénicité des conjugués étudiés et de conjugués aux molécules de comparaison.

Les résultats figurent dans le tableau II qui suit. Il fait apparaître la remarquable absence de pyrogénicité des conjugués selon l'invention, comparativement aux pyrogénicités soit du muramylpeptide lui-même, lorsque celui-ci est à l'état isolé, soit d'un conjugué que le même muramylpeptide peut former avec un antigène natif, tel que la toxine tétanique. Le conjugué selon l'invention [(LH-RH)-MTP] n'induit pas d'élévation significative de température dans l'animal, même lorsque la dose relative de MTP dans le conjugué est de l'ordre de 9,3 µg de MTP. Ces résultats sont à comparer en particulier avec ceux obtenus dans les mêmes conditions avec le conjugué de comparaison TT-MTP, lequel induit une élévation de température tout à fait considérable, même lorsque la proportion relative de MTP dans le conjugué administré est 100 fois plus réduite que dans celui de l'invention.

Il est d'ailleurs à noter en outre que des doses plus élevées du conjugué (LH-RH)-MTP contenant une proportion relative de 77 µg de MTP n'ont pas induit chez l'animal une élévation de température marquée.

TABLEAU II

| Traitement | Dose (µg) | Différences de températures individuelles (Δt) | Moyennes |
|---|---|---|---|
| Contrôle PBS | | 0; 0; 0,2; 0,1 | 0,1 |
| Contrôle LH–RH | 0,86 | 0; 0; 0,6 | 0,2 |
| | 8,6 | 0,3; 0,3; 0,5 | 0,4 |
| | 145 | 0; 0,1; 0,1 | 0,1 |
| Toxine tétanique «T–T» | 100 | 0,5; 0,1 | 0,2 |
| (LH–RH)–MTP | 0,4∼0,2 | 0; 0,2 | 0,1 |
| | 1,2∼0,6 | 0; 0,2 | 0,1 |
| | 3,4∼1,8 | 0,1; 0,1; 0,6 | 0,2 |
| | 17,2∼9,3 | 0,1; 0,3; 0,7 | 0,4 |
| | 86∼46 | 0,3 | 0,3 |
| MTP | 86 | 1,1; 1,3 | 1,2 |
| (T–T)–MTP | 100∼0,8 | 1,9; 2,1 | 2 |
| | 100∼0,7 | 1,2; 1,5 | 1,4 |

Le signe «∼» se rapporte au couplage dans les conjugués testés, entre les doses relatives dans ces conjugués, d'une part de l'hormone, d'autre part soit du MTP, soit de la toxine tétanique, ces doses étant respectivement indiquées par les premier et second nombres d'une même ligne, dans la colonne sous «Dose (µg)» du tableau présenté dans la page précédente.

II – Description de la préparation de l'ester n-butylique de

6-0-(succinylamidopropyl-2-0-(x-L-fucopyranosyl)-3-0-(α-D-galactopyrasonyl)-α-D-galactopyranosyl)-2-acétamido-2-déoxy-3-0(D-2-propionyl-L-alanyl-D-glutamine)-D-glucopyranose de formule:

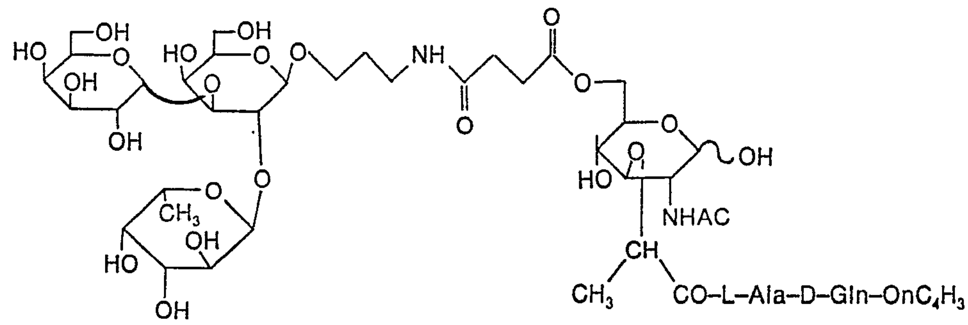

a) Préparation du
2-allyloxyéthyl 2-0-(2,3,4-tri-0-benzyl-α-L-fucopyranosyl)-3-0-(2,3,4,6-tétra-D-benzyl-α-D-galactopyranosyl)-4,6-0-benzylidène-galactopyranoside.

984 mg (0,777 mmol) de
2-hydroxyéthyl-2-0-(2,3,4-tri-0-benzyl-α-L-fucopyranosyl)-3-0-(2,3,4,6-tétra-0-benzyl-α-D-galactopyranosyl)-4,6-0-benzylidène-D-galactopyranoside
(décrit dans le brevet français FR-A-2 422 621) sont dissous dans du DMF anhydre (15 ml), puis de l'hydrure de sodium (dispersion à 50% dans l'huile; 150 mg; 3,1 mol) est ajouté. Après 1 heure, du bromure d'allyle est ajouté (0,25 ml; 2,9 mmol). Une nouvelle addition de bromure d'allyle est faite après 2 heures (0,25 ml; 2,9 mmol). Après 4 heures, du méthanol est ajouté (3 ml). Le mélange réactionnel est concentré à sec, repris dans du chloroforme qui est lavé avec une solution de chlorure de sodium saturé, avec de l'eau, puis séché sur sulfate de sodium. Après évaporation, le résidu est purifié par chromatographie sur colonne de gel de silice (30 g) dans le mélange acétate d'éthyle-hexane (1/2; v/v).

Après évaporation et séchage, une mousse blanche est obtenue: 745 mg (74,2%) $[\alpha]_D^{20} = +3°$ (c = 1, chloroforme). Le produit (I) cristallise dans l'éthanol et fond à 103–104 °C.

b) Préparation du 3-hydroxypropyl
2-0-(2,3,4-tri-0-benzyl-α-L-fucopyranosyl)-3-0-(2,3,4,6-tétra-0-benzyl-α-D-galactopyranosyl)-4,6-0-benzylidène-β-D-galactopyranoside (II).

753 mg (0,58 mmol) du produit (I) sont dissous dans du tétrahydrofuranne anhydre. A ce mélange agité sous courant d'azote, est ajoutée une solution 0,5 M de 9-borabicyclo [3.3.1] nonane (1,8 ml; 0,9 mmol). Après 4 heures à reflux, de l'éthanol est ajouté (0,3 ml) puis, après 10 minutes, de la soude 3 M (0,4 ml; 1,2 mmol) et de l'eau oxygénée 10 M (0,6 ml; 5,8 mmol). Après une heure à 50 °C, du dichlorométhane est ajouté (25 ml), puis du carbonate de potassium solide est introduit peu à peu sous agitation, jusqu'à ce que la solution soit claire.

Après filtration, évaporation du solvant et séchage, le résidu est purifié, par chromatographie sur colonne de silice G (30 g) dans le mélange acétate d'éthyle-hexane (2/1); v/v): 875 mg (67,3%) $[\alpha]_D^{20} = +1,5°$ (c: 1, chloroforme).

c) Préparation du
3-azidopropyl-2-0-(2,3,4-tri-0-benzyl-α-L-fucopyranosyl)-3-0-(2,3,4,6-tétra-0-benzyl-α-D-galactopyranosyl)-4,6,0-benzylidène-β-D-galactopyranoside (III).

517 mg (0,39 mmol) du produit II sont dissous dans du chloroforme sec (10 ml). De la triéthylamine (0,1 ml; 0,7 mmol), de la N-diméthyl-aminopyridine (4 mg) et du chlorure de tosyle fraîchement recristallisé (82 mg – 0,429 mmol) sont ajoutés. Après 24 heures sont de nouveau ajoutés de la triéthylamine (0,025 ml), de la N-diméthylaminopyridine (3 mg) et du chlorure de tosyle (20 mg).

Après 24 heures, du dichlorométhane est ajouté (20 ml) ainsi que de l'eau (5 ml).

Après 2 heures d'agitation, la phase organique est lavée avec de l'acide chlorhydrique 2 M, de l'eau, du bicarbonate de sodium en solution saturée et à l'eau.

Après séchage sur sulfate de sodium et évaporation, un résidu est obtenu (558 mg; 96,8%). Ce produit est dissous dans du diméthylformamide (15 ml), puis de l'acide de sodium (76 mg) est ajouté. Le mélange réactionnel est agité à 80 °C pendant 2 heures, puis concentré à sec. Le résidu obtenu est purifié sur colonne de gel de silice G (20 g) dans de l'acétate d'éthyle-hexane (1/1; v/v): 462 mg (87,8%). Le produit est cristallisé dans de l'éther-hexane: p.f. 86–87 °C; $[\alpha]_D^{20} = +3°$ (c = 1, chloroforme).

d) Préparation du
3-aminopropyl 2-0-(α-L-fucopyranosyl)-3-0-(α-D-galactopyranosyl)-β-D-galactopyranoside (IV).

450 mg du produit (III) sont dissous dans de l'acide acétique (30 ml) puis agités sous atmosphère d'hydrogène, en présence de palladium 5% sur charbon, pendant quatre à cinq jours. Le mélange réactionnel est filtré et le produit obtenu par lyophilisation (144,5 mg; 69%). $[\alpha]_D^{25} = +5,5°$ (c = 2, eau).

e) Préparation de la composition de formule I.

L'ester n-butylique du
6-0-succinyl-2-acétamido-2-déoxy-3-0-(D-2-propionyl-L-alanyl-D-glutamine)-D-glucopyranose
(6-0-Suc-MDPG-OnBu) (181,6 mg; 0,28 mmol) est dissous dans du diméthylformamide (6 ml). De l'hydroxybenzotriazole (48,5 mg; 0,28 mmol) et du N-cyclohexyl-N'-(β(N-méthyl-morpholino)-éthyl)

carbodiimide p-toluène sulfonate (118,5 mg; 0,28 mmol) sont ajoutés.

Après 1 heure, le produit de formule (IV) (85 mg; 0,14 mmol) est ajouté en solution dans du diméthylformamide (3 ml) en présence de N-méthylmorpholine (0,015 ml). Après 24 heures, le mélange réactionnel est concentré à sec et desséché. Le résidu obtenu est purifié sur une colonne de la résine échangeuse d'ions, connue sous la désignation AG-50- W-X2 (OH), laquelle est éluée à l'eau. Les fractions repérées par chromatographie sur couche mince sont réunies et lyophilisées. Le produit obtenu (217 mg) est purifié sur une colonne de la résine échangeuse d'ions connue sous la désignation d'AG-I-X 2 (acétate), laquelle est éluée à l'eau. Les fractions purifiées sont réunies et lyophilisées. Le produit obtenu (88 mg) est finalement purifié sur colonne de silice (colonne Lobar®, type A, Merck) éluée dans le mélange de systèmes de solvants acétate d'éthyle-pyridine-acide acétique-eau (5-5-1-3 et 6-2-0,6-1; v/v). Les fractions contenant le produit I sont réunies et lyophilisées: 47 mg $[\alpha]_D^{23} = +23,1°$ (c = 1,03 eau).

Le dosage des sucres (galactose et fucose) est fait selon Z. DISCHE et L.B. SHETTLES, «J. Biol. Chem.», 175 (1948) 595 et celui des acides aminés et de l'acide muramique par analyse en acides aminés, après hydrolyse acide totale (acide chlorhydrique 6N, 110 °C, 20 heures) selon S. MOORE et W.H. STEIN, «J. Biol. Chem.», 176 (1948) 367.

Le rapport trouvé entre la teneur en acides aminés et celle en galactose et fucose est de 0,95.

Le conjugué finalement obtenu est dépourvu de pyrogénicité. A une dose de 0,5 mg/kg, il n'induit qu'une élévation de température non significative de 0,1 °C chez le lapin.

**Revendications pour les Etats contractants BE, CH, DE, FR, GB, IT, LI, LU, NL, SE**

1. Conjugué ayant un effet immunogène entre un haptène possédant au moins un site antigénique et un muramyl-peptide, ou dérivé de muramyl-peptide, caractérisé par l'absence de macromolécule support, ce conjugué étant encore davantage caractérisé en ce que la partie haptène présente un poids moléculaire inférieur à 5000 pour annuler l'effet pyrogène qui est susceptible d'être observé à l'occasion du couplage de muramyl-peptides sur des antigènes de poids moléculaire plus élevé et, qu'en outre, ce conjugué présente un poids moléculaire inférieur à 5000 pour que le conjugué obtenu ne soit pas apte à induire en sus de l'immunogénicité attendue, la production d'anticorps dirigés contre la partie muramyl-peptide elle-même du conjugué, avec la condition supplémentaire que l'haptène est différent de l'eicosapeptide C-terminal de la béta-hCG.

2. Conjugué selon la revendication 1, caractérisé en ce qu'il n'induit pas un accroissement de température de 0,6 °C, lorsqu'il est administré par voie intraveineuse à des lapins, à raison de doses pouvant atteindre 100 microgrammes par kilo, voire 300 et même 500 microgrammes de conjugués par kilo.

3. Conjugué selon la revendication 1, caractérisé en ce que son poids moléculaire est inférieur à 3000.

4. Conjugué selon la revendication 1, caractérisé en ce que son poids moléculaire est inférieur à 2000.

5. Conjugué selon la revendication 1, caractérisé en ce que la partie haptène est un peptide qui comprend de préférence moins de 50, notamment moins de 30, de préférence moins de 20 résidus aminoacyle.

6. Conjugué selon la revendication 1, caractérisé en ce que la partie haptène est un peptide qui comprend de préférence moins de 15 résidus aminoacyle.

7. Conjugué selon l'une quelconque des revendications 1 à 6, caractérisé en ce que l'haptène a un élément de structure en commun avec un antigène natif et en ce qu'il possède un effet immunogène se manifestant par la production in vivo d'anticorps ou d'une réaction spécifique à médiation cellulaire, non seulement contre lui-même, mais en outre contre l'antigène natif lui-même.

8. Conjugué selon la revendication 7, caractérisé en ce que l'haptène possède des éléments de structure communs avec des antigènes peptidiques ou non exclusivement peptidiques possédant un pouvoir pathogène.

9. Conjugué selon l'une quelconque des revendications 1 à 6, caractérisé en ce que l'haptène est constitué par tout ou partie d'une hormone, telle que LH-RH.

10. Conjugué selon l'une quelconque des revendications 1 à 9, caractérisé en ce que la partie muramyl-peptide conjuguée à l'haptène est avantageusement dérivée du muramyl-peptide qui répond à la formule générale (I)

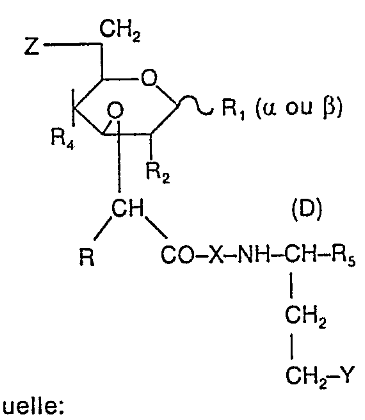

(I)

dans laquelle:

– $R_5$ est une fonction carboxyle libre ou une fonction amidée, ou une fonction carboxyle estérifiée par un alcool aliphatique possédant de 1 à 10 atomes de carbone, ou une fonction carboxyle substituée par un acide aminé possédant à son tour une fonction carboxyle libre, amidée ou estérifiée comme sus-indiqué,

– R est un hydrogène ou un méthyle,

– $R_2$ est un groupe acétamido ou glycolyl-amido,

– $R_1$ est soit $-NH_2$, soit OH, soit un radical comprenant au plus 10 atomes de carbone, correspondant à un groupe alkyle, aryle, arylalkyle ou alkylaryle, portant le cas échéant des substituants hydroxyle, amine, carboxamide, sulfonamide, éther, thioéther, ester ou thioester,

– $R_4$ est un hydroxyle ou un groupe oxyacyle comprenant de 1 à 4 atomes de carbone, notamment un groupe oxyacétyle,

– X est un résidu aminoacyle du groupe comprenant:

alanyle, arginyle, asparagyle, aspartyle, cystéinyle, glutaminyle, glutamyle, glycyle, histidyle, hydroxyprolyle, isoleucyle, leucyle, lysyle, méthionyle, phénylalanyle, prolyle, séryle, thréonyle, tryptophanyle, tyrosyle, valyle et aminobutyryle,

– Z est un groupement substituant la position en C-6 de l'acide muramique, qui répond à la formule générale (II) suivante:

$$-R_6-(R_7)_x-H \qquad (II)$$

dans laquelle x = 0 ou 1 et dans laquelle

si x = 0,

– $R_6$–H et un groupe hydroxyle ou amine et

si x = 1,

– $R_6$ est une liaison oxygène ou NH, carboxamide, sulfonamide, éther ou thioéther, ester ou thioester,

– $R_7$ étant alors un enchaînement de groupes $-CH_2-$, $-CH=CH-$, $-S-$, $-CO-$ ou $-NH-$, les groupes $-CH_2-$; $-CH=CH-$, ou $-NH-$ pouvant éventuellement être remplacés ou substitués par des groupes aryle, de préférence phényle, ou hétérocyclique comme ci-dessous définis, ou encore être substitués par des fonctions carboxylique, amino, sulfhydryle, ou hydroxyle, ou par des groupes alkyle susceptibles de comporter jusqu'à 5 atomes de carbone ou des groupes alkylaryle, arylalkyle comportant en tout jusqu'à 8 atomes de carbone ou par des groupes hétérocycliques comportant jusqu'à six atomes de carbone et un ou deux atomes d'oxygène, d'azote ou de soufre, les groupes alkylaryle ou arylalkyle susdits pouvant contenir des groupes carboxyle ou encore être substitués par des groupes carboxyle, amino, sulfhydryle ou hydroxyle et les susdits groupes hétérocycliques pouvant eux-mêmes être substitués par des fonctions alkyle pouvant contenir jusqu'à 5 atomes de carbone, carboxyle, amino, sulfhydryle ou hydroxyle, le susdit groupe $R_7$ ayant néanmoins une longueur ne dépassant pas celle d'une chaîne hydrocarbonée n-décylique,

– Y est une fonction carboxyle libre ou amidée, ou ester comprenant jusqu'à 10 atomes de carbone, ou substituée par un acide aminé, de préférence un acide aminé trifonctionnel, c'est-à-dire comportant, en sus d'une fonction carboxyle et d'une fonction amine, une fonction supplémentaire carboxyle, amine ou hydroxyle, notamment la lysine, l'ornithine, l'acide glutamique, l'acide aspartique ou la tyrosine; ou Y est un groupement qui répond à la formule générale III:

$$-R_8-(R_9)_y-H \qquad (III)$$

dans laquelle y est égal à zéro ou à 1 et dans laquelle $R_8$–H est un groupe amide ou ester, lorsque y est égal à zéro, ou lorsque y est différent de zéro, $R_8$ est une liaison carboxamide, ester ou thioester, $R_9$ ayant alors la même signification que $R_7$.

11. Conjugué selon la revendication 10, caractérisé en ce que x est un résidu glycyle, séryle, valyle, leucyle, aminobutyryle, ou de façon encore davantage préférée alanyle.

12. Conjugué selon la revendication 10 ou la revendication 11, caractérisé en ce que, x étant égal à zéro, Z est une fonction hydroxyle ou amine.

13. Conjugué selon la revendication 10 ou la revendication 11, caractérisé en ce que:

– x = 1,

– $R_6$ est une liaison carboxamide ou sulfonamide, éther ou thioéther, ester ou thioester, et

– $R_7$ représente l'un des groupements tels que ceux indiqués ci-dessous:

– $(CH_2)_n-COOH$, notamment avec n = 1, 2, 3 ou 4

– $(CH_2)_n-NH_2$, notamment avec n = 1, 2, 3 ou 4

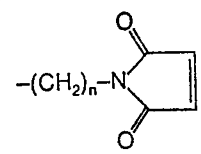

notamment avec n = 1, 2, 3 ou 4

– $(CH_2)_n-C_6H_5-NH_2$, notamment avec n = 0 ou 1

– $(CH_2)_n-SH$, notamment avec n = 2.

14. Conjugué selon l'une quelconque des revendications 10 à 13, caractérisé en ce que le groupe $R_5$ est une fonction carboxyle estérifiée par un alcool comprenant de 1 à 5 atomes de carbone, de préférence par l'alcool n-butylique, et, de préférence, Y est un groupe $-CONH_2$.

15. Conjugué selon l'une quelconque des revendications 10 à 14, caractérisé en ce que le groupe R du groupement muramyl-peptide est un groupe méthyle.

16. Conjugué selon l'une quelconque des revendications 10 à 15, caractérisé en ce que le groupe Z de la partie muramyl-peptide est un groupe succinyle.

17. Conjugué selon l'une quelconque des revendications 10 à 16, caractérisé en ce que dans la partie muramyl-peptide, $R_1$ et $R_4$ sont simultanément des hydroxyles et $R_2$ est un groupe acétamido et, de préférence, également simultanément, $R_6$ est un hydroxyle, R est un groupe méthyle, X est un groupe alanyle, $R_5$ est un groupe carboxamide ou un groupe ester comportant de 1 à 5 atomes de carbone, et Y est une fonction carboxamide.

18. Conjugué selon la revendication 10, caractérisé en ce que la partie muramyl-peptide est dérivée de l'un des muramyl-peptides suivants:

– de la N-acétyl-muramyl-L-alanyl-D-isoglutamine,

– de l'α-n-butyl-ester de la N-acétyl-muramyl-L-alanyl-D-glutamine,

– ou encore des dérivés 6-0-succinylés des deux muramyl-peptides précédents,

– d'un mono-α- ou γ-ester ou d'un diester de l'acide N-acétyl-muramyl-L-alanyl-D-glutamique

(MDPA), les groupes ester contenant de 1 à 5 atomes de carbone.

19. Conjugué selon la revendication 10, caractérisé en ce que dans la partie muramyl-peptide le groupe Y comporte un groupe lysyle et, à titre particulier, notamment en ce que la partie muramyl-peptide est dérivée de la N-acétyl-muramyl-L-alanyl-D-isoglutaminyl-L-lysine.

20. Composition pharmaceutique, caractérisée en ce qu'elle contient un conjugué conforme à l'une quelconque des revendications 1 à 19, en association avec un excipient pharmaceutiquement acceptable.

21. Composition pharmaceutique, selon la revendication 20 pour induire, au niveau de l'organisme humain ou animal, une modification de son comportement tendant à la suppression d'une hormone déterminée, caractérisée en ce que l'haptène entrant dans la composition dudit conjugué consiste en ladite hormone ou un fragment de celle-ci.

**Revendications pour l'Etat contractant AT**

1. Procédé de fabrication d'un conjugué ayant un effet immunogène entre un haptène différent de l'eicosapeptide C-terminal de la bêta-hCG, possédant au moins un site antigénique et un muramyl-peptide, ou dérivé de muramyl-peptide, caractérisé par l'absence de macromolécule support, caractérisé en ce que l'on fait réagir l'haptène avec un muramyl-peptide comprenant au moins une fonction carboxyle, ester, alcool, sulfhydryle ou amine, apte à intervenir dans le couplage covalent avec une fonction complémentaire correspondante de l'haptène, et que l'haptène présente un poids moléculaire inférieur à 5000 pour annuler l'effet pyrogène qui est susceptible d'être observé à l'occasion du couplage de muramyl-peptides sur des antigènes de poids moléculaire plus élevé et, en outre, pour que ce conjugué présente un poids moléculaire inférieur à 5000 pour que le conjugué obtenu ne soit pas apte à l'induire en sus de l'immunogénicité attendue, la production d'anticorps dirigés contre la partie muramyl-peptide elle-même du conjugué.

2. Procédé selon la revendication 1, caractérisé en ce que le poids moléculaire de l'haptène est inférieur à 3000.

3. Procédé selon la revendication 1, caractérisé en ce que le poids moléculaire de l'haptène est inférieur à 2000.

4. Procédé selon la revendication 1, caractérisé en ce que l'haptène est un peptide qui comprend de préférence moins de 50, notamment moins de 30, de préférence moins de 20 résidus aminoacyle.

5. Procédé selon la revendication 1, caractérisé en ce que l'haptène est un peptide qui comprend de préférence moins de 15 résidus aminoacyle.

6. Procédé selon l'une quelconque des revendications 1 à 5, caractérisé en ce que l'haptène a un élément de structure en commun avec un antigène natif et en ce qu'il possède un effet immunogène se manifestant par la production in vivo d'anticorps ou d'une réaction spécifique à médiation cellulaire, non seulement contre lui-même, mais en outre contre l'antigène natif lui-même.

7. Procédé selon la revendication 6, caractérisé en ce que l'haptène possède des éléments de structure communs avec des antigènes peptidiques ou non exclusivement peptidiques possédant un pouvoir pathogène.

8. Procédé selon l'une quelconque des revendications 1 à 6, caractérisé en ce que l'haptène est constitué par tout ou partie d'une hormone, telle que LH-RH.

9. Procédé selon l'une quelconque des revendications 1 à 8, caractérisé en ce que le muramyl-peptide répond à la formule générale (I):

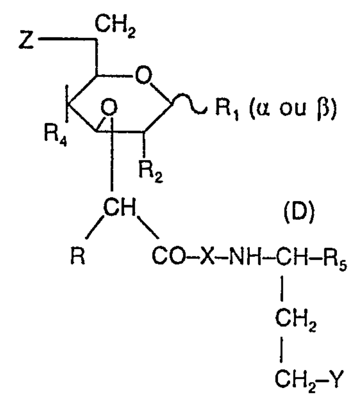
(I)

dans laquelle:

$R_5$ est une fonction carboxyle libre ou une fonction amidée, ou une fonction carboxyle estérifiée par un alcool aliphatique possédant de 1 à 10 atomes de carbone, ou une fonction carboxyle substituée par un acide aminé possédant à son tour une fonction carboxyle libre, amidée ou estérifiée comme sus-indiqué,

– R est un hydrogène ou un méthyle,

– $R_2$ est un groupe acétamido ou glycolyl-amido,

– $R_1$ est soit $-NH_2$, soit OH, soit un radical comprenant au plus 10 atomes de carbone, correspondant à un groupe alkyle, aryle, arylalkyle ou alkylaryle, portant le cas échéant des substituants hydroxyle, amine, carboxamide, sulfonamide, éther, thioéther, ester ou thioester,

– $R_4$ est un hydroxyle ou un groupe oxyacyle comprenant de 1 à 4 atomes de carbone, notamment un groupe oxyacétyle,

– X est un résidu aminoacyle du groupe comprenant:

alanyle, arginyle, asparagyle, aspartyle, cystéinyle, glutaminyle, glutamyle, glycyle, histidyle, hydroxyprolyle, isoleucyle, leucyle, lysyle, méthionyle, phénylalanyle, prolyle, séryle, thréonyle, tryptophanyle, tyrosyle, valyle et aminobutyryle,

– Z est un groupement substituant la position en C-6 de l'acide muramique, qui répond à la formule générale (II) suivante:

$$-R_6-(R_7)_x-H \qquad (II)$$

dans laquelle x = 0 ou 1 et dans laquelle

si y = 0,

– $R_6$-H est un groupe hydroxyle ou amine et

si x = 1,

– $R_6$ est une liaison oxygène ou NH, carboxamide, sulfonamide, éther ou thioéther, ester ou thioester,

– $R_7$ étant alors un enchaînement de groupes $-CH_2-$, $-CH=CH-$, -S-, -CO- ou -NH-, les groupes $-CH_2-$; $-CH=CH-$, ou -NH- pouvant éventuellement être remplacés ou substitués par des groupes aryle, de préférence phényle, ou hétérocyclique comme ci-dessous définis, ou encore être substitués par des fonctions carboxylique, amino, sulfhydryle ou hydroxyle, ou par des groupes alkyle susceptibles de comporter jusqu'à 5 atomes de carbone ou des groupes alkylaryle, arylalkyle comportant en tout jusqu'à 8 atomes de carbone ou par des groupes hétérocycliques comportant jusqu'à six atomes de carbone et un ou deux atomes d'oxygène, d'azote ou de soufre, les groupes alkylaryle ou arylalkyle susdits pouvant contenir des groupes carbonyle ou encore être substitués par des groupes carboxyle, amino, sulfhydryle ou hydroxyle et les susdits groupes hétérocycliques pouvant eux-mêmes être substitués par des fonctions alkyle pouvant contenir jusqu'à 5 atomes de carbone, carboxyle, amino, sulfhydryle ou hydroxyle, le susdit groupe $R_7$ ayant néanmoins une longueur ne dépassant pas celle d'une chaîne hydrocarbonée n-décylique,

– Y est une fonction carboxyle libre ou amidée, ou ester comportant jusqu'à 10 atomes de carbone, ou substituée par un acide aminé, de préférence un acide aminé trifonctionnel, c'est-à-dire comportant, en sus d'une fonction carboxyle et d'une fonction amine, une fonction supplémentaire carboxyle, amine ou hydroxyle, notamment la lysine, l'ornithine, l'acide glutamique, l'acide aspartique ou la tyrosine; ou Y est un groupement qui répond à la formule générale III:

$$-R_8-(R_9)_y-H \qquad \text{(III)}$$

dans laquelle y est égal à zéro ou à 1 et dans laquelle $R_8$–H est un groupe amide ou ester, lorsque y est égal à zéro, ou lorsque y est différent de zéro, $R_8$ est une liaison carboxamide, ester ou thioester, $R_9$ ayant alors la même signification que $R_7$.

10. Procédé selon la revendication 9, caractérisé en ce que x est un résidu glycyle, séryle, valyle, leucyle, aminobutyryle, ou de façon encore davantage préférée alanyle.

11. Procédé selon la revendication 9 ou la revendication 10, caractérisé en ce que, x étant égal à zéro, Z est une fonction hydroxyle ou amine.

12. Procédé selon la revendication 9 ou la revendication 10, caractérisé en ce que:

– x = 1,

– $R_6$ est une liaison carboxamide ou sulfonamide éther ou thioéther, ester ou thioester, et

– $R_7$ représente l'un des groupements tels que ceux indiqués ci-dessous:

– $(CH_2)_n-$ COOH, notamment avec n = 1, 2, 3 ou 4

– $(CH_2)_n-NH_2$, notamment avec n = 1, 2, 3 ou 4

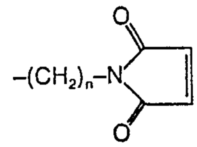

notamment avec n = 1, 2, 3 ou 4

– $(CH_2)_n-C_6H_5-NH_2$, notamment avec n = 0 ou 1

– $(CH_2)_n-$ S H, notamment avec n = 2.

13. Procédé selon l'une quelconque des revendications 9 à 12, caractérisé en ce que le groupe $R_5$ est une fonction carboxyle estérifié par un alcool comprenant de 1 à 5 atomes de carbone, de préférence par l'alcool n-butylique, et, de préférence, Y est un groupe $-CONH_2$.

14. Procédé selon l'une quelconque des revendications 9 à 13, caractérisé en ce que le groupe R du muramyl-peptide est un groupe méthyle.

15. Procédé selon l'une quelconque des revendications 9 à 14, caractérisé en ce que le groupe Z du muramyl-peptide est un groupe succinyle.

16. Procédé selon l'une quelconque des revendications 9 à 15, caractérisé en ce que dans le muramyl-peptide, $R_1$ et $R_4$ sont simultanément des hydroxyles et $R_2$ est un groupe acétamido et, de préférence, également simultanément, $R_6$ est un hydroxyle, R est un groupe méthyle, X est un groupe alanyle, $R_5$ est un groupe carboxamide ou un groupe ester comportant de 1 à 5 atomes de carbone, et Y est une fonction carboxamide.

17. Procédé selon la revendication 9, caractérisé en ce que le muramyl-peptide est dérivé de l'un des muramyl-peptides suivants:

– de la N-acétyl-muramyl-L-alanyl-D-isoglutamine,

– de l'α-n-butyl-ester de la N-acétyl-muramyl-L-alanyl-D-glutamine,

– ou encore des dérivés 6-0-succinylés des deux muramyl-peptides précédents,

– d'un mono-α- ou γ-ester ou d'un diester de l'acide N-acétyl-muramyl-L-alanyl-D-glutamique (MDPA), les groupes ester contenant de 1 à 5 atomes de carbone.

18. Procédé selon la revendication 9, caractérisé en ce que dans la partie muramyl-peptide le groupe Y comporte un groupe lysyle et, à titre particulier, notamment en ce que la partie muramyl-peptide est dérivée de la N-acétyl-muramyl-L-alanyl-D-isoglutaminyl-L-lysine.

**Claims for the contracting States BE, CH, DE, FR, GB, IT, LI, LU, NL, SE**

1. Conjugate having an immunogenic effect between a haptene possessing at least one antigenic site and a muramyl-peptide or derivative of muramyl-peptide, characterized by the absence of macromolecule carrier, said conjugate being further characterized in that the haptene part presents a molecular weight less than 5000 for annulling the pyrogenic effect which is likely to be observed at the occasion of coupling of muramylpeptides on antigenes of higher molecular weight and, furthermore, in that said conjugate presents a molecular a weight lower than 5000 in order that the conjugate obtained not be able to induce beyond the expected immunogenicity, the production of antibodies directed against the muramyl-peptide part itself of the conjugate with the additional condition that the haptene be different from the C-terminal eicosapeptide of β-hCG.

2. The conjugate of claim 1, characterized in that it does not induce an increase in temperature of 0.6 °C when administrated intravenously at a dose of 100 µg/kg in rabbits, even of 300 and even further of 500 µg/kg of conjugate per kilogram.

3. The conjugate of claim 1, characterized in that its molecular weight is less than 3000.

4. The conjugate of claim 1, characterized in that its molecular weight is less than 2000.

5. The conjugate of claim 1, characterized in that the haptene part is a peptide which comprises preferably less than 50 aminoacyl, e.g. less than 30, preferably less than 20 aminoacyl residues.

6. The conjugate of claim 1, characterized in that the haptene part is a peptide which comprises less than 15 aminoacyl residues.

7. The conjugate according to anyone of claims 1 to 6, characterized in that the haptene has a structure element in common with a native antigen and in that it possesses an immunogenic effect which manifests itself by the production in vivo of antibodies or by a specific cell mediated reaction not only against itself but also against the native antigen itself.

8. The conjugate according to claim 7, characterized in that the haptene possesses structure elements in common with peptidic or non exclusively peptidic antigens which possess a pathogenic power.

9. The conjugate according to anyone of claims 1 to 6, characterized in that the haptene is constituted by all or part of a hormone, such as LH-RH.

10. The conjugate according to anyone of claims 1 to 9, characterized in that muramyl-peptide part conjugated to the haptene is advantageously derived from the muramyl-peptide which has the general formula (I)

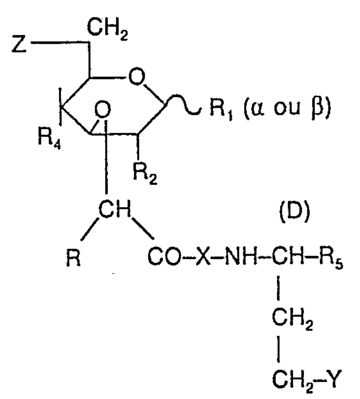

(I)

in which:

– $R_5$ is a free carboxyl group or an amido group, or a carboxyl group esterified by an aliphatic group possessing from 1 to 10 carbon atoms, or a carboxyl group substituted by an aminoacid possessing itself a carboxyl group which is either free, or amidated or esterified as indicated above,

– R is hydrogen or a methyl group,

– $R_2$ is an acetamido or a glycolyl-amido group,

– $R_1$ is either $-NH_2$, or OH, or a radical comprising at the most 10 carbon atoms, corresponding to an alkyl, aryl, arylalkyl or alkylaryl group, optionally bearing hydroxyl-, amido-, carboxamido-, sulfonamido-, ether-, thioether-, ester- or thioester substituents;

– $R_4$ is a hydroxyl or an oxyacyl group comprising from 1 to 4 carbon atoms, particularly an oxyacetyl group,

– X is an aminoacyl residue of the group comprising:

alanyl, arginyl, asparagyl, aspartyl, cysteinyl, glutaminyl, glutamyl, glycyl, histidyl, hydroxyprolyl, isoleucyl, leucyl, lysyl, methionyl, phenylalanyl, prolyl, seryl, threonyl, tryptophanyl, tyrosyl, valyl and aminobutyryl,

– Z is a group substituting the C-6 position of the muramic acid, and which has the following general formula (II):

$$-R_6-(R_7)_x-H \qquad (II)$$

in which x = 0 or 1 and in which,

if x = 0,

– $R_6$–H is hydroxyl or amino and

if x = 1,

– $R_6$ is an oxygen or NH bond, carboxamide, sulfonamide, ether or thioether, ester or thioester,

– $R_7$ then being a chain of $-CH_2-$, $-CH=-CH-$, $-S-$, $-CO-$ or NH– groups, $-CH_2$; said $-CH=CH-$, or NH-groups being optionally replaced or substituted by aryl preferably phenyl groups, or by heterocyclic groups as defined below, or by carboxylic, amino, sulfhydryl or hydroxyl groups, or by alkyl groups including up to 5 carbon atoms or by alkylaryl, arylalkyl groups including in all up to 8 carbon atoms or by heterocyclic groups including up to six carbon atoms and one or two oxygen, nitrogen or sulfur atoms, said alkylaryl or arylalkyl groups optionally containing carbonyl groups or being optionally substituted by carboxyl, amino, sulfhydryl or hydroxyl groups, said heterocyclic groups being themselves optionally substituted by alkyl groups which can contain up to 5 carbon atoms, carboxyl, amino, sulfhydryl or hydroxyl, the above said $R_7$ group having nonetheless a length no exceeding that of an n-decyl hydrocarbon chain,

– Y is a free or amidated carboxyl group, or an ester group comprising up to 10 carbon atoms, or substituted by an amino acid or is a group of formula III:

$$R_8-(R_9)_y-H \qquad (III)$$

in which y equals 0 or 1 and

in which $R_8$–H is an amide or ester group when y is equal to zero, or when y is different from zero, $R_8$ is a carboxamido-, ester- or thioester-group, $R_9$ then having the same meaning as $R_7$.

11. The conjugate of claim 10, characterized in that X is a glycyl, seryl, valyl, leucyl, aminobutyryl residue, or still more preferably alanyl.

12. The conjugate of claim 10 or claim 11, characterized in that X being equal to zero, Z is a hydroxyl or amine function.

13. The conjugate of claim 10 or claim 11, characterized in that:
- $x = 1$,
- $R_6$ is a carboxamide or sulphonamide, ether or thioether, ester or thioester linkage, and
- $R_7$ represents one of the groups such as those indicated below,
- $(CH_2)_n$–COOH, particularly with n = 1, 2, 3 or 4
- $(CH_2)_n$–$NH_2$, particularly with n = 1, 2, 3 or 4

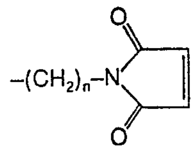

particularly with n = 1, 2, 3 or 4
- $(CH_2)_n$–$C_6H_5$–$NH_2$, particularly with n = 0 or 1
- $(CH_2)_n$–S H, particularly with n = 2.

14. The conjugate of anyone of claims 10 to 13, characterized in that the $R_5$ group is a carboxyl function esterified by an alcohol comprising from 1 to 5 carbon atoms, preferably n-butylic alcohol and, preferably Y is a $-CONH_2$ group.

15. The conjugate of anyone of claims 10 to 14, characterized in that the group R of the muramyl-peptide group is a methyl group.

16. The conjugate of anyone of claims 10 to 15, characterized in that the group Z of the muramyl-peptide part is a succinyl group.

17. The conjugate of anyone of claims 10 to 16, characterized in that, in the muramyl-peptide part, $R_1$ and $R_4$ are simultaneously hydroxyls and $R_2$ is an acetamido group and preferably too, $R_6$ is an hydroxyl, R is a methyl group, X is an alanyl group, $R_5$ is a carboxamide group or an ester group including from 1 to 5 carbon atoms, and Y is a carboxamide function.

18. The conjugate of claim 10, wherein the muramyl-peptide part is selected from one of the following muramyl-peptides:
- N-acetyl-muramyl-L-alanyl-D-isoglutamine,
- α-n-butyl-ester of N-acetyl-muramyl-L-alanyl-D-glutamine,
- or further 6-0-succinylated derivatives of the two foregoing muramyl-peptides,
- a mono-α- or γ-ester or a diester of N-acetyl-muramyl-L-alanyl-D-glutamic acid (MDPA), wherein said ester groups contain from 1 to 5 carbon atoms.

19. The conjugate of claim 10, characterized in that in the muramyl-peptide part Y includes a lysyl group and, particularly, in that the muramyl-peptide part is derived from N-acetyl-muramyl-L-alanyl-D-isoglutaminyl-L-lysine.

20. Pharmaceutical composition, characterized in that it contains a conjugate according to anyone of claims 1 to 19, in association with a pharmaceutically acceptable excipient.

21. Pharmaceutical composition according to claim 20, for inducing, at the level of the human or animal organism, a modification of its behavior tending to suppress a predetermined hormone, characterized in that the haptene portion of said conjugate consists of said hormone o a fragment thereof.

**Claims for the contracting State AT**

1. A process for the production of a conjugate having an immunogenic effect between a haptene different of the C-terminal eicosapeptide of β-hCG, possessing at least one antigenic site and a muramyl-peptide or derivative of muramyl-peptide, characterized by the absence of macromolecule carrier, characterized in that on react the haptene with a muramyl-peptide comprising at least one carboxyl-, ester-, alcohol-, sulfhydryl-, or amine function which is capable of being brought into play with a corresponding complementary function of the haptene, and in that the haptene presents a molecular weight less than 5000 for annulling the pyrogenic effect which is likely to be observed at the occasion of coupling of muramyl-peptides on antigens of higher molecular weight and, furthermore, in that said conjugate presents a molecular a weight lower than 5000 in order that the conjugate obtained not be able to induce beyond the expected immunogenicity, the production of antibodies directed against the muramyl-peptide part itself of the conjugate.

2. The process of claim 1, characterized in that its molecular weight is less than 3000.

3. The process of claim 1, characterized in that its molecular weight is less than 2000.

4. The process of claim 1, characterized in that the haptene part is a peptide which comprises preferably less than 50 aminoacyl, e.g. less than 30, preferably less than 20 aminoacyl residues.

5. The process of claim 1, characterized in that the haptene part is a peptide which comprises less than 15 aminoacyl residues.

6. The process according to anyone of claims 1 to 5, characterized in that the haptene has a structure element in common with a native antigen and in that it possesses an immunogenic effect which manifests itself by the production in vivo of antibodies or by a specific cell mediated reaction not only against itself but also against the native antigen itself.

7. The process according to claim 6, characterized in that the haptene possesses structure elements in common with peptidic or non exclusively peptidic antigens which possess a pathogenic power.

8. The process according to anyone of claims 1 to 6, characterized in that the haptene is constituted by all or part of a hormone, such as LH-RH.

9. The process according to anyone of claims 1 to 8, characterized in that muramyl-peptide part constituted to the haptene is advantageously derived from the muramyl-peptide which has the general formula (I)

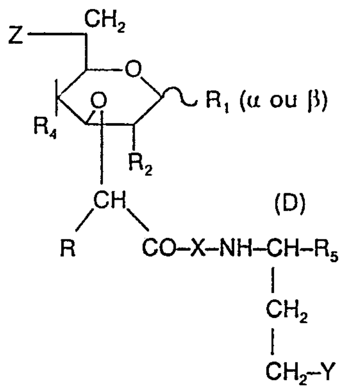

(I)

in which:

– $R_5$ is a free carboxyl group or an amido group, or a carboxyl group esterified by an aliphatic group possessing from 1 to 10 carbon atoms, or a carboxyl group substituted by an aminoacid possessing itself a carboxyl group which is either free, or amidated or esterified as indicated above,

– R is hydrogen or a methyl group,

– $R_2$ is an acetamido or a glycolyl-amido group,

– $R_1$ is either $-NH_2$, or OH, or a radical comprising at the most 10 carbon atoms, corresponding to an alkyl, aryl, arylalkyl or alkylaryl group, optionally bearing hydroxyl-, amido-, carboxamido-, sulfonamido-, ether-, thioether-, ester- or thioester substituents;

– $R_4$ is a hydroxyl or an oxyacyl group comprising from 1 to 4 carbon atoms, particularly an oxyacetyl group,

– X is an aminoacyl residue of the group comprising:

alanyl, arginyl, asparagyl, aspartyl, cysteinyl, glutaminyl, glutamyl, glycyl, histidyl, hydroxyprolyl, isoleucyl, leucyl, lysyl, methionyl, phenylalanyl, prolyl, seryl, threonyl, tryptophanyl, tyrosyl, valyl and aminobutyryl,

– Z is a group substituting the C-6 position of the muramic acid, and which has the following general formula (II):

$$-R_6-(R_7)_x-H \qquad (II)$$

in which x = 0 or 1 and in which,

if x = 0,

– $R_6$–H is hydroxyl or amino and

if x = 1,

– $R_6$ is an oxygen or NH bond, carboxamide, sulfonamide, ether or thioether, ester or thioester,

–$R_7$ then being a chain of $-CH_2-$, $-CH=-CH-$, $-S-$, $-CO-$ or NH– groups, $-CH_2-$; said $-CH=CH-$, or NH– groups being optionally replaced or substituted by aryl preferably phenyl groups, or by heterocyclic groups as defined below, or by carboxylic, amino, sulfhydryl or hydroxyl groups, or by alkyl groups including up to 5 carbon atoms or by alkylaryl, arylalkyl groups including in all up to 8 carbon atoms or by heterocyclic groups including up to six carbon atoms and one or two oxygen, nitrogen or sulfur atoms, said alkylaryl or arylalkyl groups optionally containing carbonyl groups or being optionally substituted by carboxyl, amino, sulfhydryl or hydroxyl groups, said heterocyclic groups being themeselves optionally substituted by alkyl groups which can contain up to 5 carbon atoms, carboxyl, amino, sulfhydryl or hydroxyl, the above said $R_7$ group having nonetheless a length no exceeding that of an n-decyl hydrocarbon chain,

– Y is a free or amidated carboxyl group, or an ester group comprising up to 10 carbon atoms, or substituted by an amino acid or is a group of formula III:

$$R_8-(R_9)_y-H \qquad (III)$$

in which y equals 0 or 1 and in which $R_8$–H is an amide or ester group when y is equal to zero, or when y is different from zero, $R_8$ is a carboxamido-, ester- or thioester-group, $R_9$ then having the same meaning as $R_7$.

10. The process of claim 9, characterized in that X is a glycyl, seryl, valyl, leucyl, aminobutyryl residue, or still more preferably alanyl.

11. The process of claim 9 or claim 10, characterized in that X being equal to zero, Z is a hydroxyl or amine function.

12. The process of claim 9 or claim 10, characterized in that:

– x = 1,

– $R_6$ is a carboxamide or sulphonamide, ether or thioether, ester or thioester linkage, and

– $R_7$ represents one of the groups such as those indicated below,

– $(CH_2)_n$–COOH, particularly with n = 1, 2, 3 or 4

– $(CH_2)_n$–$NH_2$, particularly with n = 1, 2, 3 or 4

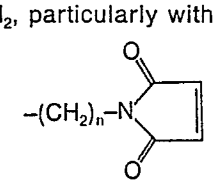

particularly with n = 1, 2, 3 or 4

– $(CH_2)_nC_6H_5$–$NH_2$, particularly with n = 0 or 1

– $(CH_2)_n$–S H, particularly with n = 2.

13. The process of anyone of claims 9 to 12, characterized in that the $R_5$ group is a carboxyl function esterified by an alcohol comprising from 1 to 5 carbon atoms, preferably n-butylic alcohol and, preferably Y is a $-CONH_2$ group.

14. The process of anyone of claims 9 to 13, characterized in that the group R of the muramyl-peptide group is a methyl group.

15. The process of anyone of claims 9 to 14, characterized in that the group Z of the muramyl-peptide part is a succinyl group.

16. The process of anyone of claims 9 to 15, characterized in that, in the muramyl-peptide part, $R_1$ and $R_4$ are simultaneously hydroxyls and $R_2$ is an acetamido group and preferably too, $R_6$ is an hydroxyl, R is a methyl group, X is an alanyl group, $R_5$ is a carboxamide group or an ester group including from 1 to 5 carbon atoms, and Y is a carboxamide function.

17. The process of claim 9, wherein the muramyl-peptide part is selected from one of the following muramyl-peptides:

– N-acetyl-muramyl-L-alanyl-D-isoglutamine,

– α-n-butyl-ester of N-acetyl-muramyl-L-alanyl-D-glutamine,

– or further 6-0-succinylated derivatives of the two foregoing muramyl-peptides,

– a mono-α- or γ-ester or a diester of N-acetyl-muramyl-L-alanyl-D-glutamic acid (MDPA), wherein said ester groups contain from 1 to 5 carbon atoms.

18. The process of claim 9, characterized in that in the muramyl-peptide part Y includes a lysyl group and, particularly, in that the muramyl-peptide part is derived from N-acetyl-muramyl-L-alanyl-D-isoglutaminyl-L-lysine.

**Patentansprüche für die Vertragsstaaten BE, CH, DE, FR, GB, IT, LI, LU, NL, SE**

1. Konjugat mit immunogener Wirkung zwischen einem Hapten, welches mindestens eine antigene Stelle aufweist, und einem Muramylpeptid oder einem Muramylpeptidderivat, gekennzeichnet durch die Abwesenweit eines makromolekularen Trägers, wobei der Konjugat weiterhin dadurch gekennzeichnet ist, daß der Haptenanteil ein Molekulargewicht unter 5000 aufweist, um pyrogene Wirkungen zu vermeiden, die bei der Koppelung von Muramylpeptiden auf Antigenen mit einem höheren Molekulargewicht beobachtet werden und daß der Komplex weiterhin ein Molekulargewicht unter 5000 aufweist, damit der erhaltene Komplex nicht dazu neigt, über der erwarteten Immunogenität hinaus die Produktion von Antikörpern zu induzieren, die gegen den Muramylpeptidteil des Konjugats selbst gerichtet sind, unter der zusätzlichen Bedingung, daß das Hapten sich vom Eicosapeptid-C-Terminal des Beta-hCG unterscheidet.

2. Komplex nach Anspruch 1, dadurch gekennzeichnet, daß er einen Temperaturanstieg von 0,6 °C nicht hervorruft, wenn er intravenös an Kaninchen verabreicht wird, wobei die Dosierungen bis zu 100 Mikrogramm pro Kilo, 300 und selbst 500 Mikrogramm Komplex pro Kilo betragen können.

3. Konjugat nach Anspruch 1, dadurch gekennzeichnet, daß sein Molekulargewicht geringer als 3000 ist.

4. Konjugat nach Anspruch 1, dadurch gekennzeichnet, daß sein Molekulargewicht geringer als 2000 ist.

5. Konjugat nach Anspruch 1, dadurch gekennzeichnet, daß der Haptenanteil ein Peptid ist, das vorzugsweise weniger als 50, insbesondere weniger als 30, vorzugsweise weniger als 20 Aminosäurereste enthält.

6. Konjugat nach Anspruch 1, dadurch gekennzeichnet, daß der Haptenanteil ein Peptid ist, das vorzugsweise weniger als 15 Aminosäurereste enthält.

7. Konjugat nach einem der Ansprüche 1 bis 6, dadurch gekennzeichnet, daß das Hapten ein Strukturelement gemeinsam mit einem ursprünglichen Antigen hat und daß es eine immunogene Wirkung aufweist, die sich durch die in vivo Produktion von Antikörpern oder durch eine spezifische Reaktion der Zellmediation nicht nur gegen das Hapten sondern unter anderem auch gegen das ursprüngliche Antigen zeigt.

8. Konjugat nach Anspruch 8, dadurch gekennzeichnet, daß das Hapten gemeinsame Strukturelemente mit den peptidischen oder nicht ausschließlich peptidischen Antigenen, die eine pathogene Wirkung haben, aufweist.

9. Konjugat nach einem der Ansprüche 1 bis 6, dadurch gekennzeichnet, daß das Hapten ganz oder zu einem Teil aus einem Hormon, wie LH-RH, besteht.

10. Konjugat nach einem der Ansprüche 1 bis 6, dadurch gekennzeichnet, daß der mit dem Hapten konjugierte Muramyl-Peptid-Teil vorteilhafterweise von einem Muramyl-Peptid abgeleitet ist, das der allgemeinen Formel (I) entspricht

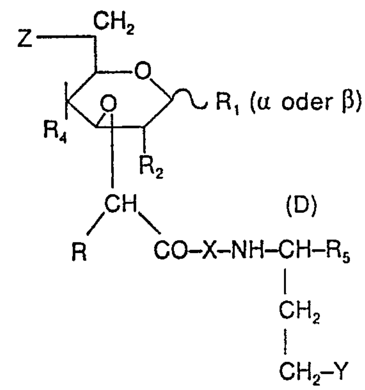

(I)

worin:

– $R_5$ eine freie oder eine amidierte Carboxylgruppe oder eine Carboxylgruppe, die durch einen aliphatischen Alkohol mit 1 bis 10 Kohlenstoffatomen verestert ist, oder eine Carboxylgruppe ist, die durch eine Aminosäure, die ihrerseits eine freie, amidierte oder veresterte Carboxylgruppe wie oben angegeben besitzt, substituiert ist,

– R ein Wasserstoff oder ein Methyl ist,

– $R_2$ eine Acetamido- oder Glykolylamidogruppe ist,

– $R_1$ entweder $-NH_2$ oder OH oder eine Alkyl-, Aryl-, Arylalkyl- oder Alkylarylgruppe mit höchstens 10 Kohlenstoffatomen, die gegebenenfalls Hydroxyl-, Amin-, Carboxamid-, Sulfonamid-, Ether-, Thioether-, Ester- oder Thioestersubstituenten trägt, ist

– $R_4$ Hydroxyl oder eine Oxyacylgruppe mit 1 bis 4 Kohlenstoffatomen, insbesondere eine Oxyacetylgruppe, ist,

– X ein Aminosäurerest aus der Gruppe Alanyl, Arginyl, Asparagyl, Aspartyl, Cysteinyl, Glutaminyl, Glutamyl, Glycyl, Histidyl, Hydroxyprolyl, Isoleucyl, Leucyl, Lysyl, Methionyl, Phenylalanyl, Prolyl, Seryl, Threonyl, Tryptophanyl, Tyrosyl, Valyl und Aminobutyryl ist,

– Z eine Gruppe ist, die die Position in C-6 der Muraminsäure substituiert, die der folgenden allgemeinen Formel (II) entspricht:

$$-R_6-(R_7)_x-H \qquad (II)$$

worin x = 0 oder 1 ist und worin, wenn x = 0 ist,

– $R_6$–H eine Hydroxyl- oder Amingruppe ist und wenn x = 1 ist,

– $R_6$ eine Sauerstoff- oder NH-, Carboxamid-, Sulfonamid-, Ether- oder Thioether-, Ester- oder Thioester-Brücke ist,

– $R_7$ dann eine Kette aus den Gruppen $-CH_2-$, $-CH=CH-$, $-S-$, $-CO-$ oder $-NH-$ ist, wobei die Gruppen $-CH_2-$; $-CH=CH-$, oder $-NH-$ eventuell durch Arylgruppen ersetzt oder substituiert sind, vorzugsweise durch Phenylgruppen oder heterocyclische Gruppen wie unten definiert, oder die durch Carboxyl-, Amino-, Sulfhydryl- oder Hydroxylgruppen oder durch Alkylgruppen, die bis zu 5 Kohlenstoffatome aufzuweisen, oder durch Alkylaryl-, Arylalkylgruppen mit insgesamt bis zu 8 Kohlenstoffatomen oder durch heterocyclische Gruppen mit bis zu 6 Kohlenstoffatomen und ein oder zwei Sauerstoff-, Stickstoff- oder Schwefelatomen enthalten, wobei die oben genannten Alkylaryl- oder Arylalkylgruppen Carboxylgruppen enthalten können oder aber durch Carboxyl-, Amino-, Sulfhydryl- oder Hydroxylgruppen substituiert sein können und die oben genannten heterocyclischen Gruppen selbst durch Alkylfunktionen mit bis zu 5 Kohlenstoffatomen, Carboxyl-, Amino-, Sulfhydryl- oder Hydroxylfunktionen substituiert sein können, wobei die oben genannte Gruppe $R_7$ nichtsdestoweniger eine Länge aufweist, die diejenige einer n-Decyl-Kohlenwasserstoff-Kette nicht übersteigt,

– Y eine freie oder amidierte Carboxylgruppe oder ein Ester ist, der bis zu 10 Kohlenstoffatome aufweist oder durch eine Aminosäure substituiert ist, vorzugsweise durch eine trifunktionale Aminosäure, d.h. die zusätzlich zu einer Carboxylgruppe und einer Amingruppe eine zusätzliche Carboxyl-, Amin- oder Hydroxylgruppe aufweist, insbesondere Lysin, Ornithin, Glutaminsäure, Asparaginsäure oder Tyrosin; oder Y eine Gruppe ist, die der allgemeinen Formel III entspricht:

$$-R_8-(R_9)_y-H \qquad (III)$$

worin y 0 oder 1 ist und
worin $R_8$–H eine Amidgruppe oder ein Ester ist, wenn y gleich Null ist oder wenn y verschieden von Null ist, $R_8$ eine Carboxamid-, Ester- oder Thioesterbrücke ist, wobei $R_9$ dann die gleiche Bedeutung wie $R_7$ hat.

11. Konjugat nach Anspruch 10, dadurch gekennzeichnet, daß X ein Glycyl-, Seryl-, Valyl-, Leucyl-, Aminobutyrylrest oder vorzugsweise ein Alanylrest ist.

12. Konjugat nach Anspruch 10 oder Anspruch 11, dadurch gekennzeichnet, daß x gleich Null ist und Z eine Hydroxyl- oder Aminfunktion ist.

13. Konjugat nach Anspruch 10 oder 11, dadurch gekennzeichnet, daß

– $x = 1$,

– $R_6$ eine Carboxamid- oder Sulfonamidbrücke, Ether- oder Thioether-, Ester- oder Thioester-Brücke ist, und

– $R_7$ eine der Gruppen ist, wie sie nachstehend aufgeführt sind:

– $(CH_2)_n-COOH$, insbesondere mit $n = 1, 2, 3$ oder 4

– $(CH_2)_n-CH_2$, insbesondere mit $n = 1, 2, 3$ oder 4

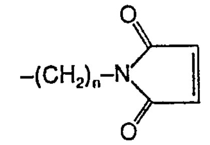

insbesondere mit $n = 1, 2, 3$ oder 4

– $(CH_2)_n-C_6H_5-NH_2$, insbesondere mit $n = 0$ oder 1

– $(CH_2)_n-SH$, insbesondere mit $n = 2$.

14. Konjugat nach einem der Ansprüche 10 bis 13, dadurch gekennzeichnet, daß die Gruppe $R_5$ eine Carboxylgruppe ist, die durch einen Alkohol mit 1 bis 5 Kohlenstoffatomen, vorzugsweise durch n-Butylalkohol, verestert ist und vorzugsweise Y eine $-CONH_2$-Gruppe ist.

15. Konjugat nach einem der Ansprüche 10 bis 14, dadurch gekennzeichnet, daß die R-Gruppe der Muramyl-Peptid-Gruppe eine Methylgruppe ist.

16. Konjugat nach einem der Ansprüche 10 bis 15, dadurch gekennzeichnet, daß die Z-Gruppe des Muramyl-Peptid-Teils eine Succinylgruppe ist.

17. Konjugat nach einem der Ansprüche 10 bis 16, dadurch gekennzeichnet, daß in dem Muramyl-Peptid-Teil, $R_1$ und $R_4$ gleichzeitig Hydroxyl sind und $R_2$ eine Acetamidogruppe ist und, vorzugsweise, ebenfalls gleichzeitig, $R_6$ ein Hydroxyl, R eine Methylgruppe, X eine Alanylgruppe, $R_5$ eine Carboxamidgruppe oder eine Estergruppe mit 1 bis 5 Kohlenstoffatomen und Y eine Carboxamidgruppe ist.

18. Konjugat nach Anspruch 10, dadurch gekennzeichnet, daß der Muramyl-Peptid-Teil von einem der folgenden Muramyl-Peptide abgeleitet ist:

– von N-Acetyl-muramyl-L-alanyl-D-isoglutamin,

– von α-n-Butyl-ester des N-Acetyl-muramyl-L-alanyl-D-glutamins,

– oder von 6-0-succinylierten Derivaten der beiden voranstehenden Muramyl-Peptide,

– eines Mono-α- oder γ-Esters oder eines Diesters der N-Acetyl-muramyl-L-alanyl-D-glutaminsäure (MDPA), wobei die Estergruppen 1 bis 5 Kohlenstoffatome enthalten.

19. Konjugat nach Anspruch 10, dadurch gekennzeichnet, daß im Muramyl-Peptid-Teil die Y-Gruppe eine Lysylgruppe enthält und insbesondere dadurch, daß der Muramyl-Peptid-Teil von N-Acetyl-muramyl-L-analyl-D-isoglutaminyl-L-lysin abgeleitet ist.

20. Pharmazeutisches Mittel, dadurch gekennzeichnet, daß es ein Konjugat nach einem der Ansprüche 1 bis 19 in Verbindung mit einem pharmazeutisch verträglichen Träger enthält.

21. Pharmazeutisches Mittel nach Anspruch 20, welches in einem menschlichen oder tierischen Organismus eine Veränderung seines Verhaltens bewirkt, das zur Unterdrückung eines bestimmten Hormons führt, dadurch gekennzeichnet, daß das in dem genannten Konjugat vorhandene Hapten

aus dem genannten Hormon oder einem Fragment desselben besteht.

**Patentansprüche für den Vertragsstaat AT**

1. Verfahren zur Herstellung eines Konjugats mit immunogener Wirkung zwischen einem Hapten, das sich vom Eicosapeptid-C-Terminal des Beta-hCG unterscheidet, das mindestens eine antigene Stelle aufweist, und einem Muramylpeptid oder einem Muramylpeptidderivat, gekennzeichnet durch die Abwesenheit eines makromolekularen Trägers, dadurch gekennzeichnet, daß man das Hapten mit einem Muramylpeptid mit mindestens einer Carboxyl-, Ester-, Alkohol-, Sulfhydryl- oder Amingruppe, die geeignet ist, bei der kovalenten Koppelung mit einer entsprechenden komplementären Gruppe des Haptens zu intervenieren, umsetzt und daß das Hapten ein Molekulargewicht von weniger als 5000 aufweist, um pyrogene Wirkungen zu vermeiden, die bei der Koppelung von Muramylpeptiden auf Antigenen mit einem höheren Molekulargewicht beobachtet werden und daß das Konjugat weiterhin ein Molekulargewicht unter 5000 aufweist, damit das erhaltene Konjugat nicht dazu neigt, über der erwarteten Immunogenität hinaus die Produktion von Antikörpern zu induzieren, die gegen den Muramylpeptidteil des Konjugats selbst gerichtet sind.

2. Verfahren nach Anspruch 1, dadurch gekennzeichnet, daß das Molekulargewicht des Haptens geringer als 3000 ist.

3. Verfahren nach Anspruch 1, dadurch gekennzeichnet, daß das Molekulargewicht des Haptens geringer als 2000 ist.

4. Verfahren nach Anspruch 1, dadurch gekennzeichnet, daß das Hapten ein Peptid ist, das vorzugsweise weniger als 50, insbesondere weniger als 30, vorzugsweise weniger als 20 Aminosäurereste enthält.

5. Verfahren nach Anspruch 1, dadurch gekennzeichnet, daß das Hapten ein Peptid ist, das vorzugsweise weniger als 15 Aminosäurereste enthält.

6. Verfahren nach einem der Ansprüche 1 bis 5, dadurch gekennzeichnet, daß das Hapten ein Strukturelement gemeinsam mit einem ursprünglichen Antigen hat und daß es eine immunogene Wirkung aufweist, die sich durch die in vivo Produktion von Antikörpern oder durch eine spezifische Reaktion der Zellmediation nicht nur gegen das Hapten sondern unter anderem auch gegen das ursprüngliche Antigen zeigt.

7. Verfahren nach Anspruch 6, dadurch gekennzeichnet, daß das Hapten gemeinsame Strukturelemente mit den peptidischen oder nicht ausschließlich peptidischen Antigenen, die eine pathogene Wirkung haben, aufweist.

8. Verfahren nach einem der Ansprüche 1 bis 6, dadurch gekennzeichnet, daß das Hapten ganz oder zu einem Teil aus einem Hormon, wie LH-RH, besteht.

9. Verfahren nach einem der Ansprüche 1 bis 8, dadurch gekennzeichnet, daß das Muramyl-Peptid der allgemeinen Formel (I) entspricht:

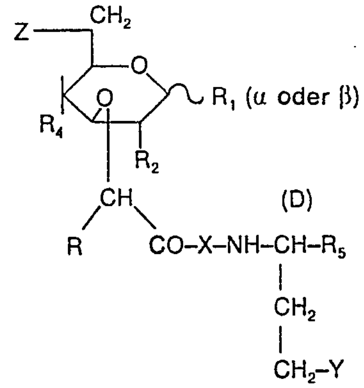

(I)

worin:

– $R_5$ eine freie oder eine amidierte Carboxylgruppe oder eine Carboxylgruppe, die durch einen aliphatischen Alkohol mit 1 bis 10 Kohlenstoffatomen verestert ist, oder eine Carboxylgruppe ist, die durch eine Aminosäure, die ihrerseits eine freie, amidierte oder veresterte Carboxylgruppe wie oben angegeben besitzt, substituiert ist,

– R ein Wasserstoff oder ein Methyl ist,

– $R_2$ eine Acetamido- oder Glykolylamidogruppe ist,

– $R_1$ entweder $-NH_2$ oder OH oder eine Alkyl-, Aryl-, Arylalkyl- oder Alkylarylgruppe mit höchstens 10 Kohlenstoffatomen, die gegebenenfalls Hydroxyl-, Amin-, Carboxamid-, Sulfonamid-, Ether-, Thioether-, Ester- oder Thioestersubstituenten trägt, ist,

– $R_4$ Hydroxyl oder eine Oxyacylgruppe mit 1 bis 4 Kohlenstoffatomen, insbesondere eine Oxyacetylgruppe, ist,

– X ein Aminosäurerest aus der Gruppe Alanyl, Arginyl, Asparagyl, Aspartyl, Cysteinyl, Glutaminyl, Glutamyl, Glycyl, Histidyl, Hydroxyprolyl, Isoleucyl, Leucyl, Lysyl, Methionyl, Phenylalanyl, Prolyl, Seryl, Threonyl, Tryptophanyl, Tyrosyl, Valyl und Aminobutyryl ist,

– Z eine Gruppe ist, die die Position in C-6 der Muraminsäure substituiert, die der folgenden allgemeinen Formel (II) entspricht:

$$-R_6-(R_7)_x-H \qquad (II)$$

worin x = 0 oder 1 ist und worin, wenn x = 0 ist,

– $R_6$–H eine Hydroxyl- oder Amingruppe ist und wenn x = 1 ist,

– $R_6$ eine Sauerstoff- oder NH-, Carboxamid-, Sulfonamid-, Ether- oder Thioether-, Ester- oder Thioester-Brücke ist,

– $R_7$ dann eine Kette aus den Gruppen $-CH_2-$, $-CH{=}CH-$, $-S-$, $-CO-$ oder $-NH-$ ist, wobei die Gruppen $-CH_2-$; $-CH{=}CH-$, oder $-NH-$ eventuell durch Arylgruppen ersetzt oder substituiert sind, vorzugsweise durch Phenylgruppen oder heterocyclische Gruppen wie unten definiert, oder die durch Carboxyl-, Amino-, Sulfhydryl- oder Hydroxylgruppen oder durch Alkylgruppen, die bis zu 5 Kohlenstoffatome aufzuweisen, oder durch Alkylaryl-, Arylalkylgruppen mit insgesamt bis zu 8 Kohlenstoffatomen oder durch heterocyclische Gruppen mit bis zu 6 Kohlenstoffatomen und die ein oder zwei Sauerstoff-, Stickstoff- oder Schwefelatomen enthalten, wobei die oben genannten Alkylaryl- oder Arylalkylgruppen Carbonylgruppen enthalten können oder aber durch Carboxyl-, Amino-, Sulfhydryl- oder Hydroxylgruppen substituiert sein können und die oben genannten heterocyclischen Gruppen selbst durch Alkylfunktionen mit bis zu 5 Kohlenstoffatomen, Carboxyl-, Amino-, Sulfhydryl- oder Hydroxylfunktionen substituiert sein können, wobei die oben genannte Gruppe $R_7$ nichtsdestoweniger eine Länge aufweist, die diejenige einer n-Decyl-Kohlenwasserstoff-Kette nicht übersteigt,

– Y eine freie oder amidierte Carboxylgruppe oder ein Ester ist, der bis zu 10 Kohlenstoffatome aufweist oder durch eine Aminosäure substituiert ist, vorzugsweise durch eine trifunktionale Aminosäure, d.h. die zusätzlich zu einer Carboxylgruppe und einer Amingruppe eine zusätzliche Carboxyl-, Amin- oder Hydroxylgruppe aufweist, insbesondere Lysin, Ornithin, Glutaminsäure, Asparaginsäure oder Tyrosin; oder Y eine Gruppe ist, die der allgemeinen Formel III entspricht:

$$-R_8-(R_9)_y-H \qquad (III)$$

worin y 0 oder 1 ist und

worin $R_8$–H eine Amidgruppe oder ein Ester ist, wenn y gleich Null ist oder wenn y verschieden von Null ist, $R_8$ eine Carboxamid-, Ester- oder Thioesterbrücke ist, wobei $R_9$ dann die gleiche Bedeutung wie $R_7$ hat.

10. Verfahren nach Anspruch 9, dadurch gekennzeichnet, daß X ein Glycyl-, Seryl-, Valyl-, Leucyl-, Aminobutyrylrest oder vorzugsweise ein Alanylrest ist.

11. Verfahren nach Anspruch 9 oder Anspruch 10, dadurch gekennzeichnet, daß x gleich Null ist und Z eine Hydroxyl- oder Aminfunktion ist.

12. Verfahren nach Anspruch 9 oder 10, dadurch gekennzeichnet, daß

– x = 1,

– $R_6$ eine Carboxamid- oder Sulfonamidbrücke, Ether- oder Thioether-, Ester- oder Thioester-Brücke ist, und

– $R_7$ eine der Gruppen ist, wie sie nachstehend aufgeführt sind:

– $(CH_2)_n$–COOH, insbesondere mit n = 1, 2, 3 oder 4

– $(CH_2)_n$–$NH_2$, insbesondere mit n = 1, 2, 3 oder 4

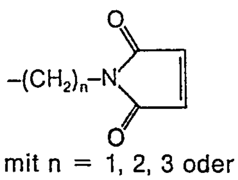

insbesondere mit n = 1, 2, 3 oder 4

– $(CH_2)_n$–$C_6H_5$–$NH_2$, insbesondere mit n = 0 oder 1

– $(CH_2)_n$–SH, insbesondere mit n = 2.

13. Verfahren nach einem der Ansprüche 9 bis 12, dadurch gekennzeichnet, daß die Gruppe $R_5$ eine Carboxylgruppe ist, die durch einen Alkohol mit 1 bis 5 Kohlenstoffatomen, vorzugsweise durch n-Butylalkohol, verestert ist und vorzugsweise Y eine –$CONH_2$-Gruppe ist.

14. Verfahren nach einem der Ansprüche 9 bis 13, dadurch gekennzeichnet, daß die R-Gruppe der Muramyl-Peptid-Gruppe eine Methylgruppe ist.

15. Verfahren nach einem der Ansprüche 9 bis 14, dadurch gekennzeichnet, daß die Z-Gruppe des Muramyl-Peptids eine Succinylgruppe ist.

16. Verfahren nach einem der Ansprüche 9 bis 15, dadurch gekennzeichnet, daß in dem Muramyl-Peptid, $R_1$ und $R_4$ gleichzeitig Hydroxyl sind und $R_2$ eine Acetamidogruppe ist und, vorzugsweise, ebenfalls gleichzeitig, $R_6$ ein Hydroxyl, R eine Methylgruppe, X eine Alanylgruppe, $R_5$ eine Carboxamidgruppe oder eine Estergruppe mit 1 bis 5 Kohlenstoffatomen und Y eine Carboxamidgruppe ist.

17. Verfahren nach Anspruch 9, dadurch gekennzeichnet, daß das Muramyl-Peptid von einem der folgenden Muramyl-Peptide abgeleitet ist:

– von N-Acetyl-muramyl-L-alanyl-D-isoglutamin,

– von α-n-Butyl-ester des N-Acetyl-muramyl-L-alanyl-D-glutamins,

– oder von 6-0-succinylierten Derivaten der beiden voranstehenden Muramyl-Peptide,

– eines Mono-α- oder γ-Esters oder eines Diesters der N-Acetyl-muramyl-L-alanyl-D-glutaminsäure (MDPA), wobei die Estergruppen 1 bis 5 Kohlenstoffatome enthalten.

18. Verfahren nach Anspruch 9, dadurch gekennzeichnet, daß im Muramyl-Peptid-Teil die Y-Gruppe eine Lysylgruppe enthält und insbesondere dadurch, daß der Muramyl-Peptid-Teil von N-Acetyl-muramyl-L-alanyl-D-isoglutaminyl-L-lysin abgeleitet ist.